# EUROPEAN PATENT SPECIFICATION

(11) **EP 3 017 824 B1**
(45) Date of publication and mention of the grant of the patent: **05.06.2019**
(21) Application number: 14782228.2
(22) Date of filing: 08.04.2014
(51) Int. Cl.: A61K 38/16, A23L 33/10, A61K 38/40, A23L 33/19, A61P 7/00, A61P 9/10, A61P 13/12, A61P 37/02, A61P 43/00

(54) **INHIBITOR OF EXTRACELLULAR TRAP FORMATION IN LEUKOCYTES**
INHIBITOR DER BILDUNG VON EXTRAZELLULÄREN FALLEN IN LEUKOZYTEN
INHIBITEUR DE LA FORMATION DE PIÈGES EXTRACELLULAIRES DANS LES LEUCOCYTES

(30) Priority: 09.04.2013 JP 2013081243
(43) Date of publication of application: 11.05.2016
(73) Proprietor: The University of Tokyo, Tokyo 113-8654 (JP); NRL Pharma, Inc., Takatsu-ku Kawasaki-shi, Kanagawa 213-0012 (JP)
(72) Inventor: HIRAHASHI, Junichi, Tokyo 113-8654 (JP); URANO, Yasuteru, Tokyo 113-8654 (JP); OKUBO, Koushu, Tokyo 113-8654 (JP); KAMIYA, Mako, Tokyo 113-8654 (JP); KAGAYA, Shinji, Kawasaki-shi Kanagawa 213-0012 (JP)
(74) Representative: Appleyard Lees IP LLP
(86) International application number: PCT/JP2014/060561
(87) International publication number: WO 2014/168253

(56) References cited:
- WO-A2-2007/022537
- JP-A- 2001 048 804
- JP-A- 2011 524 748
- US-A1- 2007 142 292
- BOURNAZOU, I. ET AL.: 'Apoptotic human cells inhibit migration of granulocytes via release of lactoferrin' THE JOURNAL OF CLINICAL INVESTIGATION vol. 119, no. 1, 2009, pages 20 - 32, XP002552533
- CAIELLI, S. ET AL.: 'Neutrophils come of age in chronic inflammation' CURRENT OPINION IN IMMUNOLOGY vol. 24, no. 6, 2012, pages 671 - 677, XP055285148

### Sequence listing

### SEQUENCE LISTING

<110> NRL Pharma The University of Tokyo
<120> Leukocyte Extracellular Trap Inhibitor
<130> G1004WO
<150> JP 2013-081243
   <151> 2013-04-09
<160> 5
<170> PatentIn version 3.5
<210> 1
   <211> 666
   <212> PRT
   <213> Homo sapiens
<400> 1
   Met Arg Lys Val Arg Gly Pro Pro Val Ser Cys Ile Lys Arg Asp Ser
   1               5                   10                  15
   Pro Ile Gln Cys Ile Gln Ala Ile Ala Glu Asn Arg Ala Asp Ala Val
               20                  25                  30
   Thr Leu Asp Gly Gly Phe Ile Tyr Glu Ala Gly Leu Ala Pro Tyr Lys
           35                  40                  45
   Leu Arg Pro Val Ala Ala Glu Val Tyr Gly Thr Glu Arg Gln Pro Arg
       50                  55                  60
   Thr His Tyr Tyr Ala Val Ala Val Val Lys Lys Gly Gly Ser Phe Gln
   65 70 75 80
   Leu Asn Glu Leu Gln Gly Leu Lys Ser Cys His Thr Gly Leu Arg Arg
   85 90 95
   Thr Ala Gly Trp Asn Val Pro Ile Gly Thr Leu Arg Pro Phe Leu Asn
   100 105 110
   Trp Thr Gly Pro Pro Glu Pro Ile Glu Ala Ala Val Ala Arg Phe Phe
   115 120 125
   Ser Ala Ser Cys Val Pro Gly Ala Asp Lys Gly Gln Phe Pro Asn Leu
   130 135 140
   Cys Arg Leu Cys Ala Gly Thr Gly Glu Asn Lys Cys Ala Phe Ser Ser
   145 150 155 160
   Gln Glu Pro Tyr Phe Ser Tyr Ser Gly Ala Phe Lys Cys Leu Arg Asp
   165 170 175
   Gly Ala Gly Asp Val Ala Phe Ile Arg Glu Ser Thr Val Phe Glu Asp
   180 185 190
   Leu Ser Asp Glu Ala Glu Arg Asp Glu Tyr Glu Leu Leu Cys Pro Asp
   195 200 205
   Asn Thr Arg Lys Pro Val Asp Lys Phe Lys Asp Cys His Leu Ala Arg
   210 215 220
   Val Pro Ser His Ala Val Val Ala Arg Ser Val Asn Gly Lys Glu Asp
   225 230 235 240
   Ala Ile Trp Asn Leu Leu Arg Gln Ala Gln Glu Lys Phe Gly Lys Asp
   245 250 255
   Lys Ser Pro Lys Phe Gln Leu Phe Gly Ser Pro Ser Gly Gln Lys Asp
   260 265 270
   Leu Leu Phe Lys Asp Ser Ala Ile Gly Phe Ser Arg Val Pro Pro Arg
   275 280 285
   Ile Asp Ser Gly Leu Tyr Leu Gly Ser Gly Tyr Phe Thr Ala Ile Gln
   290 295 300
   Asn Leu Arg Lys Ser Glu Glu Glu Val Ala Ala Arg Arg Ala Arg Val
   305 310 315 320
   Val Trp Cys Ala Val Gly Glu Gln Glu Leu Arg Lys Cys Asn Gln Trp
   325 330 335
   Ser Gly Leu Ser Glu Gly Ser Val Thr Cys Ser Ser Ala Ser Thr Thr
   340 345 350
   Glu Asp Cys Ile Ala Leu Val Leu Lys Gly Glu Ala Asp Ala Met Ser
   355 360 365
   Leu Asp Gly Gly Tyr Val Tyr Thr Ala Gly Lys Cys Gly Leu Val Pro
   370 375 380
   Val Leu Ala Glu Asn Tyr Lys Ser Gln Gln Ser Ser Asp Pro Asp Pro
   385 390 395 400
   Asn Cys Val Asp Arg Pro Val Glu Gly Tyr Leu Ala Val Ala Val Val
   405 410 415
   Arg Arg Ser Asp Thr Ser Leu Thr Trp Asn Ser Val Lys Gly Lys Lys
   420 425 430
   Ser Cys His Thr Ala Val Asp Arg Thr Ala Gly Trp Asn Ile Pro Met
   435 440 445
   Gly Leu Leu Phe Asn Gln Thr Gly Ser Cys Lys Phe Asp Glu Tyr Phe
   450 455 460
   Ser Gln Ser Cys Ala Pro Gly Ser Asp Pro Arg Ser Asn Leu Cys Ala
   465 470 475 480
   Leu Cys Ile Gly Asp Glu Gln Gly Glu Asn Lys Cys Val Pro Asn Ser
   485 490 495
   Asn Glu Arg Tyr Tyr Gly Tyr Thr Gly Ala Phe Arg Cys Leu Ala Glu
   500 505 510
   Asn Ala Gly Asp Val Ala Phe Val Lys Asp Val Thr Val Leu Gln Asn
   515 520 525
   Thr Asp Gly Asn Asn Asn Glu Ala Trp Ala Lys Asp Leu Lys Leu Ala
   530 535 540
   Asp Phe Ala Leu Leu Cys Leu Asp Gly Lys Arg Lys Pro Val Thr Glu
   545 550 555 560
   Ala Arg Ser Cys His Leu Ala Met Ala Pro Asn His Ala Val Val Ser
   565 570 575
   Arg Met Asp Lys Val Glu Arg Leu Lys Gln Val Leu Leu His Gln Gln
   580 585 590
   Ala Lys Phe Gly Arg Asn Gly Ser Asp Cys Pro Asp Lys Phe Cys Leu
   595 600 605
   Phe Gln Ser Glu Thr Lys Asn Leu Leu Phe Asn Asp Asn Thr Glu Cys
   610 615 620
   Leu Ala Arg Leu His Gly Lys Thr Thr Tyr Glu Lys Tyr Leu Gly Pro
   625 630 635 640
   Gln Tyr Val Ala Gly Ile Thr Asn Leu Lys Lys Cys Ser Thr Ser Pro
   645 650 655
   Leu Leu Glu Ala Cys Glu Phe Leu Arg Lys
   660 665
<210> 2
   <211> 708
   <212> PRT
   <213> Bos taurus
<400> 2
   Met Lys Leu Phe Val Pro Ala Leu Leu Ser Leu Gly Ala Leu Gly Leu
   1 5 10 15
   Cys Leu Ala Ala Pro Arg Lys Asn Val Arg Trp Cys Thr Ile Ser Gln
   20 25 30
   Pro Glu Trp Phe Lys Cys Arg Arg Trp Gln Trp Arg Met Lys Lys Leu
   35 40 45
   Gly Ala Pro Ser Ile Thr Cys Val Arg Arg Ala Phe Ala Leu Glu Cys
   50 55 60
   Ile Arg Ala Ile Ala Glu Lys Lys Ala Asp Ala Val Thr Leu Asp Gly
   65 70 75 80
   Gly Met Val Phe Glu Ala Gly Arg Asp Pro Tyr Lys Leu Arg Pro Val
   85 90 95
   Ala Ala Glu Ile Tyr Gly Thr Lys Glu Ser Pro Gln Thr His Tyr Tyr
   100 105 110
   Ala Val Ala Val Val Lys Lys Gly Ser Asn Phe Gln Leu Asp Gln Leu
   115 120 125
   Gln Gly Arg Lys Ser Cys His Thr Gly Leu Gly Arg Ser Ala Gly Trp
   130 135 140
   Ile Ile Pro Met Gly Ile Leu Arg Pro Tyr Leu Ser Trp Thr Glu Ser
   145 150 155 160
   Leu Glu Pro Leu Gln Gly Ala Val Ala Lys Phe Phe Ser Ala Ser Cys
   165 170 175
   Val Pro Cys Ile Asp Arg Gln Ala Tyr Pro Asn Leu Cys Gln Leu Cys
   180 185 190
   Lys Gly Glu Gly Glu Asn Gln Cys Ala Cys Ser Ser Arg Glu Pro Tyr
   195 200 205
   Phe Gly Tyr Ser Gly Ala Phe Lys Cys Leu Gln Asp Gly Ala Gly Asp
   210 215 220
   Val Ala Phe Val Lys Glu Thr Thr Val Phe Glu Asn Leu Pro Glu Lys
   225 230 235 240
   Ala Asp Arg Asp Gln Tyr Glu Leu Leu Cys Leu Asn Asn Ser Arg Ala
   245 250 255
   Pro Val Asp Ala Phe Lys Glu Cys His Leu Ala Gln Val Pro Ser His
   260 265 270
   Ala Val Val Ala Arg Ser Val Asp Gly Lys Glu Asp Leu Ile Trp Lys
   275 280 285
   Leu Leu Ser Lys Ala Gln Glu Lys Phe Gly Lys Asn Lys Ser Arg Ser
   290 295 300
   Phe Gln Leu Phe Gly Ser Pro Pro Gly Gln Arg Asp Leu Leu Phe Lys
   305 310 315 320
   Asp Ser Ala Leu Gly Phe Leu Arg Ile Pro Ser Lys Val Asp Ser Ala
   325 330 335
   Leu Tyr Leu Gly Ser Arg Tyr Leu Thr Thr Leu Lys Asn Leu Arg Glu
   340 345 350
   Thr Ala Glu Glu Val Lys Ala Arg Tyr Thr Arg Val Val Trp Cys Ala
   355 360 365
   Val Gly Pro Glu Glu Gln Lys Lys Cys Gln Gln Trp Ser Gln Gln Ser
   370 375 380
   Gly Gln Asn Val Thr Cys Ala Thr Ala Ser Thr Thr Asp Asp Cys Ile
   385 390 395 400
   Val Leu Val Leu Lys Gly Glu Ala Asp Ala Leu Asn Leu Asp Gly Gly
   405 410 415
   Tyr Ile Tyr Thr Ala Gly Lys Cys Gly Leu Val Pro Val Leu Ala Glu
   420 425 430
   Asn Arg Lys Ser Ser Lys His Ser Ser Leu Asp Cys Val Leu Arg Pro
   435 440 445
   Thr Glu Gly Tyr Leu Ala Val Ala Val Val Lys Lys Ala Asn Glu Gly
   450 455 460
   Leu Thr Trp Asn Ser Leu Lys Asp Lys Lys Ser Cys His Thr Ala Val
   465 470 475 480
   Asp Arg Thr Ala Gly Trp Asn Ile Pro Met Gly Leu Ile Val Asn Gln
   485 490 495
   Thr Gly Ser Cys Ala Phe Asp Glu Phe Phe Ser Gln Ser Cys Ala Pro
   500 505 510
   Gly Ala Asp Pro Lys Ser Arg Leu Cys Ala Leu Cys Ala Gly Asp Asp
   515 520 525
   Gln Gly Leu Asp Lys Cys Val Pro Asn Ser Lys Glu Lys Tyr Tyr Gly
   530 535 540
   Tyr Thr Gly Ala Phe Arg Cys Leu Ala Glu Asp Val Gly Asp Val Ala
   545 550 555 560
   Phe Val Lys Asn Asp Thr Val Trp Glu Asn Thr Asn Gly Glu Ser Thr
   565 570 575
   Ala Asp Trp Ala Lys Asn Leu Asn Arg Glu Asp Phe Arg Leu Leu Cys
   580 585 590
   Leu Asp Gly Thr Arg Lys Pro Val Thr Glu Ala Gln Ser Cys His Leu
   595 600 605
   Ala Val Ala Pro Asn His Ala Val Val Ser Arg Ser Asp Arg Ala Ala
   610 615 620
   His Val Lys Gln Val Leu Leu His Gln Gln Ala Leu Phe Gly Lys Asn
   625 630 635 640
   Gly Lys Asn Cys Pro Asp Lys Phe Cys Leu Phe Lys Ser Glu Thr Lys
   645 650 655
   Asn Leu Leu Phe Asn Asp Asn Thr Glu Cys Leu Ala Lys Leu Gly Gly
   660 665 670
   Arg Pro Thr Tyr Glu Glu Tyr Leu Gly Thr Glu Tyr Val Thr Ala Ile
   675 680 685
   Ala Asn Leu Lys Lys Cys Ser Thr Ser Pro Leu Leu Glu Ala Cys Ala
   690 695 700
   Phe Leu Thr Arg
   705
<210> 3
   <211> 708
   <212> PRT
   <213> Ovis aries
<400> 3
   Met Lys Leu Phe Val Pro Ala Leu Leu Ser Leu Gly Ala Leu Gly Leu
   1 5 10 15
   Cys Leu Ala Ala Pro Arg Lys Asn Val Arg Trp Cys Ala Ile Ser Pro
   20 25 30
   Pro Glu Gly Ser Lys Cys Tyr Gln Trp Gln Arg Arg Met Arg Lys Leu
   35 40 45
   Gly Ala Pro Ser Ile Thr Cys Val Arg Arg Thr Ser Ala Leu Glu Cys
   50 55 60
   Ile Arg Ala Ile Ala Gly Lys Lys Ala Asp Ala Val Thr Leu Asp Ser
   65 70 75 80
   Gly Met Val Phe Glu Ala Gly Leu Asp Pro Tyr Lys Leu Arg Pro Val
   85 90 95
   Ala Ala Glu Ile Tyr Gly Thr Glu Lys Ser Pro Gln Thr His Tyr Tyr
   100 105 110
   Ala Val Ala Val Val Lys Lys Gly Ser Asn Phe Gln Leu Asp Gln Leu
   115 120 125
   Gln Gly Gln Lys Ser Cys His Met Gly Leu Gly Arg Ser Ala Gly Trp
   130 135 140
   Asn Ile Pro Met Gly Ile Leu Arg Pro Phe Leu Ser Trp Thr Glu Ser
   145 150 155 160
   Ala Glu Pro Leu Gln Gly Ala Val Ala Arg Phe Phe Ser Ala Ser Cys
   165 170 175
   Val Pro Cys Val Asp Gly Lys Ala Tyr Pro Asn Leu Cys Gln Leu Cys
   180 185 190
   Lys Gly Val Gly Glu Asn Lys Cys Ala Cys Ser Ser Gln Glu Pro Tyr
   195 200 205
   Phe Gly Tyr Ser Gly Ala Phe Lys Cys Leu Gln Asp Gly Ala Gly Asp
   210 215 220
   Val Ala Phe Val Lys Glu Thr Thr Val Phe Glu Asn Leu Pro Glu Lys
   225 230 235 240
   Ala Asp Arg Asp Gln Tyr Glu Leu Leu Cys Leu Asn Asn Thr Arg Ala
   245 250 255
   Pro Val Asp Ala Phe Lys Glu Cys His Leu Ala Gln Val Pro Ser His
   260 265 270
   Ala Val Val Ala Arg Ser Val Asp Gly Lys Glu Asn Leu Ile Trp Glu
   275 280 285
   Leu Leu Arg Lys Ala Gln Glu Lys Phe Gly Lys Asn Lys Ser Gln Arg
   290 295 300
   Phe Gln Leu Phe Gly Ser Pro Gln Gly Gln Lys Asp Leu Leu Phe Lys
   305 310 315 320
   Asp Ser Ala Leu Gly Phe Val Arg Ile Pro Ser Lys Val Asp Ser Ala
   325 330 335
   Leu Tyr Leu Gly Ser Arg Tyr Leu Thr Ala Leu Lys Asn Leu Arg Glu
   340 345 350
   Thr Ala Glu Glu Val Lys Ala Arg Cys Thr Arg Val Val Trp Cys Ala
   355 360 365
   Val Gly Pro Glu Glu His Ser Lys Cys Gln Gln Trp Ser Glu Gln Ser
   370 375 380
   Gly Gln Asn Val Thr Cys Ala Met Ala Ser Thr Thr Asp Asp Cys Ile
   385 390 395 400
   Ala Leu Val Leu Lys Gly Glu Ala Asp Ala Leu Ser Leu Asp Gly Gly
   405 410 415
   Tyr Ile Tyr Thr Ala Gly Lys Cys Gly Leu Val Pro Val Met Ala Glu
   420 425 430
   Asn Arg Glu Ser Ser Lys Tyr Ser Ser Leu Asp Cys Val Leu Arg Pro
   435 440 445
   Thr Glu Gly Tyr Leu Ala Val Ala Val Val Lys Lys Ala Asn Glu Gly
   450 455 460
   Leu Thr Trp Asn Ser Leu Lys Gly Lys Lys Ser Cys His Thr Ala Val
   465 470 475 480
   Asp Arg Thr Ala Gly Trp Asn Ile Pro Met Gly Leu Ile Ala Asn Gln
   485 490 495
   Thr Gly Ser Cys Ala Phe Asp Glu Phe Phe Ser Gln Ser Cys Ala Pro
   500 505 510
   Gly Ala Asp Pro Lys Ser Ser Leu Cys Ala Leu Cys Ala Gly Asp Asp
   515 520 525
   Gln Gly Leu Asn Lys Cys Val Pro Asn Ser Lys Glu Lys Tyr Tyr Gly
   530 535 540
   Tyr Thr Gly Ala Phe Arg Cys Leu Ala Glu Asp Val Gly Asp Val Ala
   545 550 555 560
   Phe Val Lys Asn Asp Thr Val Trp Glu Asn Thr Asn Gly Glu Ser Ser
   565 570 575
   Ala Asp Trp Ala Lys Asn Leu Asn Arg Glu Asp Phe Arg Leu Leu Cys
   580 585 590
   Leu Asp Gly Thr Thr Lys Pro Val Thr Glu Ala Gln Ser Cys Tyr Leu
   595 600 605
   Ala Val Ala Pro Asn His Ala Val Val Ser Arg Ser Asp Arg Ala Ala
   610 615 620
   His Val Glu Gln Val Leu Leu His Gln Gln Ala Leu Phe Gly Lys Asn
   625 630 635 640
   Gly Lys Asn Cys Pro Asp Gln Phe Cys Leu Phe Lys Ser Glu Thr Lys
   645 650 655
   Asn Leu Leu Phe Asn Asp Asn Thr Glu Cys Leu Ala Lys Leu Gly Gly
   660 665 670
   Arg Pro Thr Tyr Glu Lys Tyr Leu Gly Thr Glu Tyr Val Thr Ala Ile
   675 680 685
   Ala Asn Leu Lys Lys Cys Ser Thr Ser Pro Leu Leu Glu Ala Cys Ala
   690 695 700
   Phe Leu Thr Arg
   705
<210> 4
   <211> 708
   <212> PRT
   <213> Capra hircus
<400> 4
   Met Lys Leu Phe Val Pro Ala Leu Leu Ser Leu Gly Ala Leu Gly Leu
   1 5 10 15
   Cys Leu Ala Ala Pro Arg Lys Asn Val Arg Trp Cys Ala Ile Ser Leu
   20 25 30
   Pro Glu Trp Ser Lys Cys Tyr Gln Trp Gln Arg Arg Met Arg Lys Leu
   35 40 45
   Gly Ala Pro Ser Ile Thr Cys Ile Arg Arg Thr Ser Ala Leu Glu Cys
   50 55 60
   Ile Arg Ala Ile Ala Gly Lys Asn Ala Asp Ala Val Thr Leu Asp Ser
   65 70 75 80
   Gly Met Val Phe Glu Ala Gly Leu Asp Pro Tyr Lys Leu Arg Pro Val
   85 90 95
   Ala Ala Glu Ile Tyr Gly Thr Glu Lys Ser Pro Gln Thr His Tyr Tyr
   100 105 110
   Ala Val Ala Val Val Lys Lys Gly Ser Asn Phe Gln Leu Asp Gln Leu
   115 120 125
   Gln Gly Gln Lys Ser Cys His Met Gly Leu Gly Arg Ser Ala Gly Trp
   130 135 140
   Asn Ile Pro Val Gly Ile Leu Arg Pro Phe Leu Ser Trp Thr Glu Ser
   145 150 155 160
   Ala Glu Pro Leu Gln Gly Ala Val Ala Arg Phe Phe Ser Ala Ser Cys
   165 170 175
   Val Pro Cys Val Asp Gly Lys Ala Tyr Pro Asn Leu Cys Gln Leu Cys
   180 185 190
   Lys Gly Val Gly Glu Asn Lys Cys Ala Cys Ser Ser Gln Glu Pro Tyr
   195 200 205
   Phe Gly Tyr Ser Gly Ala Phe Lys Cys Leu Gln Asp Gly Ala Gly Asp
   210 215 220
   Val Ala Phe Val Lys Glu Thr Thr Val Phe Glu Asn Leu Pro Glu Lys
   225 230 235 240
   Ala Asp Arg Asp Gln Tyr Glu Leu Leu Cys Leu Asn Asn Thr Arg Ala
   245 250 255
   Pro Val Asp Ala Phe Lys Glu Cys His Leu Ala Gln Val Pro Ser His
   260 265 270
   Ala Val Val Ala Arg Ser Val Asp Gly Lys Glu Asn Leu Ile Trp Glu
   275 280 285
   Leu Leu Arg Lys Ala Gln Glu Lys Phe Gly Lys Asn Lys Ser Gln Ser
   290 295 300
   Phe Gln Leu Phe Gly Ser Pro Glu Gly Arg Arg Asp Leu Leu Phe Lys
   305 310 315 320
   Asp Ser Ala Leu Gly Phe Val Arg Ile Pro Ser Lys Val Asp Ser Ala
   325 330 335
   Leu Tyr Leu Gly Ser Arg Tyr Leu Thr Ala Leu Lys Asn Leu Arg Glu
   340 345 350
   Thr Ala Glu Glu Leu Lys Ala Arg Cys Thr Arg Val Val Trp Cys Ala
   355 360 365
   Val Gly Pro Glu Glu Gln Ser Lys Cys Gln Gln Trp Ser Glu Gln Ser
   370 375 380
   Gly Gln Asn Val Thr Cys Ala Thr Ala Ser Thr Thr Asp Asp Cys Ile
   385 390 395 400
   Ala Leu Val Leu Lys Gly Glu Ala Asp Ala Leu Ser Leu Asp Gly Gly
   405 410 415
   Tyr Ile Tyr Thr Ala Gly Lys Cys Gly Leu Val Pro Val Met Ala Glu
   420 425 430
   Asn Arg Lys Ser Ser Lys Tyr Ser Ser Leu Asp Cys Val Leu Arg Pro
   435 440 445
   Thr Glu Gly Tyr Leu Ala Val Ala Val Val Lys Lys Ala Asn Glu Gly
   450 455 460
   Leu Thr Trp Asn Ser Leu Lys Gly Lys Lys Ser Cys His Thr Ala Val
   465 470 475 480
   Asp Arg Thr Ala Gly Trp Asn Ile Pro Met Gly Leu Ile Ala Asn Gln
   485 490 495
   Thr Gly Ser Cys Ala Phe Asp Glu Phe Phe Ser Gln Ser Cys Ala Pro
   500 505 510
   Gly Ala Asp Pro Lys Ser Ser Leu Cys Ala Leu Cys Ala Gly Asp Asp
   515 520 525
   Gln Gly Leu Asp Lys Cys Val Pro Asn Ser Lys Glu Lys Tyr Tyr Gly
   530 535 540
   Tyr Thr Gly Ala Phe Arg Cys Leu Ala Glu Asp Val Gly Asp Val Ala
   545 550 555 560
   Phe Val Lys Asn Asp Thr Val Trp Glu Asn Thr Asn Gly Glu Ser Ser
   565 570 575
   Ala Asp Trp Ala Lys Asn Leu Asn Arg Glu Asp Phe Arg Leu Leu Cys
   580 585 590
   Leu Asp Gly Thr Thr Lys Pro Val Thr Glu Ala Gln Ser Cys Tyr Leu
   595 600 605
   Ala Val Ala Pro Asn His Ala Val Val Ser Arg Ser Asp Arg Ala Ala
   610 615 620
   His Val Glu Gln Val Leu Leu His Gln Gln Ala Leu Phe Gly Lys Asn
   625 630 635 640
   Gly Lys Asn Cys Pro Asp Gln Phe Cys Leu Phe Lys Ser Glu Thr Lys
   645 650 655
   Asn Leu Leu Phe Asn Asp Asn Thr Glu Cys Leu Ala Lys Leu Gly Gly
   660 665 670
   Arg Pro Thr Tyr Glu Lys Tyr Leu Gly Thr Glu Tyr Val Thr Ala Ile
   675 680 685
   Ala Asn Leu Lys Lys Cys Ser Thr Ser Pro Leu Leu Glu Ala Cys Ala
   690 695 700
   Phe Leu Thr Arg
   705
<210> 5
   <211> 695
   <212> PRT
   <213> Equus caballus
<400> 5
   Leu Gly Leu Cys Leu Ala Ala Pro Arg Lys Ser Val Arg Trp Cys Thr
   1 5 10 15
   Ile Ser Pro Ala Glu Ala Ala Lys Cys Ala Lys Phe Gln Arg Asn Met
   20 25 30
   Lys Lys Val Arg Gly Pro Ser Val Ser Cys Ile Arg Lys Thr Ser Ser
   35 40 45
   Phe Glu Cys Ile Gln Ala Ile Ala Ala Asn Lys Ala Asp Ala Val Thr
   50 55 60
   Leu Asp Gly Gly Leu Val Tyr Glu Ala Gly Leu His Pro Tyr Lys Leu
   65 70 75 80
   Arg Pro Val Ala Ala Glu Val Tyr Gln Thr Arg Gly Lys Pro Gln Thr
   85 90 95
   Arg Tyr Tyr Ala Val Ala Val Val Lys Lys Gly Ser Gly Phe Gln Leu
   100 105 110
   Asn Gln Leu Gln Gly Val Lys Ser Cys His Thr Gly Leu Gly Arg Ser
   115 120 125
   Ala Gly Trp Asn Ile Pro Ile Gly Thr Leu Arg Pro Tyr Leu Asn Trp
   130 135 140
   Thr Gly Pro Pro Glu Pro Leu Gln Lys Ala Val Ala Asn Phe Phe Ser
   145 150 155 160
   Ala Ser Cys Val Pro Cys Ala Asp Gly Lys Gln Tyr Pro Asn Leu Cys
   165 170 175
   Arg Leu Cys Ala Gly Thr Glu Ala Asp Lys Cys Ala Cys Ser Ser Gln
   180 185 190
   Glu Pro Tyr Phe Gly Tyr Ser Gly Ala Phe Lys Cys Leu Glu Asn Gly
   195 200 205
   Ala Gly Asp Val Ala Phe Val Lys Asp Ser Thr Val Phe Glu Asn Leu
   210 215 220
   Pro Asp Glu Ala Asp Arg Asp Lys Tyr Glu Leu Leu Cys Pro Asp Asn
   225 230 235 240
   Thr Arg Lys Pro Val Asp Ala Phe Lys Glu Cys His Leu Ala Arg Val
   245 250 255
   Pro Ser His Ala Val Val Ala Arg Ser Val Asp Gly Arg Glu Asp Leu
   260 265 270
   Ile Trp Arg Leu Leu His Arg Ala Gln Glu Glu Phe Gly Arg Asn Lys
   275 280 285
   Ser Ser Ala Phe Gln Leu Phe Lys Ser Thr Pro Glu Asn Lys Asp Leu
   290 295 300
   Leu Phe Lys Asp Ser Ala Leu Gly Phe Val Arg Ile Pro Ser Gln Ile
   305 310 315 320
   Asp Ser Gly Leu Tyr Leu Gly Ala Asn Tyr Leu Thr Ala Thr Gln Asn
   325 330 335
   Leu Arg Glu Thr Ala Ala Glu Val Ala Ala Arg Arg Glu Arg Val Val
   340 345 350
   Trp Cys Ala Val Gly Pro Glu Glu Glu Arg Lys Cys Lys Gln Trp Ser
   355 360 365
   Asp Val Ser Asn Arg Lys Val Ala Cys Ala Ser Ala Ser Thr Thr Glu
   370 375 380
   Glu Cys Ile Ala Leu Val Leu Lys Gly Glu Ala Asp Ala Leu Asn Leu
   385 390 395 400
   Asp Gly Gly Phe Ile Tyr Val Ala Gly Lys Cys Gly Leu Val Pro Val
   405 410 415
   Leu Ala Glu Asn Gln Lys Ser Gln Asn Ser Asn Ala Pro Asp Cys Val
   420 425 430
   His Arg Pro Pro Glu Gly Tyr Leu Ala Val Ala Val Val Arg Lys Ser
   435 440 445
   Asp Ala Asp Leu Thr Trp Asn Ser Leu Ser Gly Lys Lys Ser Cys His
   450 455 460
   Thr Gly Val Gly Arg Thr Ala Ala Trp Asn Ile Pro Met Gly Leu Leu
   465 470 475 480
   Phe Asn Gln Thr Gly Ser Cys Lys Phe Asp Lys Phe Phe Ser Gln Ser
   485 490 495
   Cys Ala Pro Gly Ala Asp Pro Gln Ser Ser Leu Cys Ala Leu Cys Val
   500 505 510
   Gly Asn Asn Glu Asn Glu Asn Lys Cys Met Pro Asn Ser Glu Glu Arg
   515 520 525
   Tyr Tyr Gly Tyr Thr Gly Ala Phe Arg Cys Leu Ala Glu Lys Ala Gly
   530 535 540
   Asp Val Ala Phe Val Lys Asp Val Thr Val Leu Gln Asn Thr Asp Gly
   545 550 555 560
   Lys Asn Ser Glu Pro Trp Ala Lys Asp Leu Lys Gln Glu Asp Phe Glu
   565 570 575
   Leu Leu Cys Leu Asp Gly Thr Arg Lys Pro Val Ala Glu Ala Glu Ser
   580 585 590
   Cys His Leu Ala Arg Ala Pro Asn His Ala Val Val Ser Gln Ser Asp
   595 600 605
   Arg Ala Gln His Leu Lys Lys Val Leu Phe Leu Gln Gln Asp Gln Phe
   610 615 620
   Gly Gly Asn Gly Pro Asp Cys Pro Gly Lys Phe Cys Leu Phe Lys Ser
   625 630 635 640
   Glu Thr Lys Asn Leu Leu Phe Asn Asp Asn Thr Glu Cys Leu Ala Glu
   645 650 655
   Leu Gln Gly Lys Thr Thr Tyr Glu Gln Tyr Leu Gly Ser Glu Tyr Val
   660 665 670
   Thr Ser Ile Thr Asn Leu Arg Arg Cys Ser Ser Ser Pro Leu Leu Glu
   675 680 685
   Ala Cys Ala Phe Leu Arg Ala
   690 695

## Description

### TECHNICAL FIELD

The present invention relates to a pharmaceutical composition for use in treating a disease associated with formation of leukocyte extracellular traps, the composition comprising lactoferrin, wherein the disease is selected from the group consisting of microscopic polyangitis, allergic granulomatosis-angitis, local Shwartzman reaction, acute kidney injury (AKI) accompanied by ischemia reperfusion injury and disseminated intravascular coagulation.

### BACKGROUND ART

NETs (neutrophil extracellular traps) are extracellular structures that release mesh-like structures, capture bacteria, true fungi, parasitic worms and viruses, and exhibit an antibacterial action when neutrophils are activated by contamination with bacteria, the form of the segmented neutrophils and the distribution of chromatin are made unclear, then the nuclear membrane is extinguished, cytoplasm and granular components are existent in the chromatin structure in a mixed state, and the cell membrane is broken. NETs mainly involve DNAs, and histone 3 (H3) and elastase play an important role in the action of NETs. Formation of the NETs locally collects antibacterial molecules that efficiently kill microorganisms. The formation of the NETs causes NETosis of the neutrophils, but the molecular mechanism thereof has not been much clarified. The NETs are formed by the neutrophils being stimulated with TNF-α, PMA, LPS, IL-8 or the like, and NETosis caused at the time of the formation of NETs exhibits a different form from classically known necrosis or apoptosis that causes activation of caspase or fragmentation of DNAs. When the neutrophils are stimulated by LPS or PMA, autophagy is caused, and at the same time, activated oxygen is generated. This causes degradation of nuclear membrane, decondensation of chromatin, and citrullination of histone, and thus NETosis is caused (Non-patent documents 1 and 2).

It has been reported that along with the formation of the NETs, many proteins, especially, antibacterial proteins contained in azurophilic granule or type-II granule, are secreted from the neutrophils by degranulation, and proteins related to cell structure are also secreted (Non-patent document 2). These proteins are also called "NETs constructing proteins". Reported as such proteins are neutrophil elastase, histone, myeloperoxidase, F-actin, lactoferrin, matrix metalloprotease 9, LL37, cathepsin G, BPI, proteinase 3, calprotectin, azurocidin, lysozyme C, defensin, catalase and the like (Non-patent document 3).

Lactoferrin is contained in type-II granule in the neutrophils. The neutrophils form the NETs and finally cause degranulation, and lactoferrin is released to the outside of the neutrophils (Non-patent document 1).

Lactoferrin is well preserved in mammals, and there is little difference in the function of lactoferrin among different species of mammals. Today, lactoferrin is a target of attention as a protein contained in milk for the physiological activity thereof, and is commercially available. It should be noted that milk has been reported as not having an inhibitory effect on the formation of the NETs of bovine neutrophils (Non-patent document 4).

The NETs are involved in enlargement and growth of thrombus. When the NETs are released, histone contained therein has an action of condensing platelets. Thus, platelet thrombus is formed based on the NETs. Neutrophil elastase or cathepsin G contained in the NETs degrades tissue factor pathway inhibitor and promotes blood clotting reaction. The NETs play a role of keeping the microorganisms and the like at a local site by such an action (Non-patent document 5).

The action of the NETs that is to be exhibited originally is to capture external microorganisms such as gram-positive bacteria, gram-negative bacteria, true fungi and the like, and confine and kill such external microorganisms at a local site. Owing to having such an action, the NETs are often seen in infectious diseases. However, it has also been reported that when an infectious disease becomes chronic, the NETs are formed even in the absence of external microorganisms. Systemic lupus erythematosus (SLE), which is one of chronic and intractable autoimmune diseases, is known to form autoantibody against self-DNA or related proteins and thus cause inflammation in tissues or organs. A characteristic finding regarding SLE is that many neutrophils are present in the injury site. It is known that in the serum of an SLE patient, antibacterial peptide LL37 and DNAs in the NETs are existent (Non-patent documents 6 and 7). Such a substance is recognized by B cells as autoantigen, and thus autoantibody is produced. The neutrophils in an SLE patient are more likely to cause NETosis than the neutrophils in a healthy person (Non-patent document 6). These factors are considered to induce chronic inflammation. It has been reported that the IgG fragment in the serum of a patient of anti-neutrophil cytoplasmic antibody (ANCA) associated vasculitis, which is a disease caused by autoantibody, has an ability of forming the neutrophil NETs that is about twice as high as the ability of a healthy person (Non-patent documents 8 and 9).

The relationship between the formation of the NETs and diseases has been a target of attention merely recently, more specifically, since the report by Brinkman et al. in 2004 (Non-patent document 1) was made. In the future, diseases caused by the formation of the NETs will be newly revealed. Although the formation of the NETs plays an important role in protecting a living body against infection, inhibition of the formation of the NETs is considered to be necessary to improve some disease conditions as described above.

A substance that inhibits the formation of the NETs, for example, Streptococcus, which is gram-positive bacteria such as Streptococcus pneumoniae, Staphylococcus aureus or the like have been reported to express DNase extracellularly. As substances that suppress production of antibody against histone or superoxide, diphenyleneiodoniumchloride (DPI) and catalase have been reported (Non-patent document 10).

In addition, it is known, for example, that myeloperoxidase (MPO) activity influences NETosis (Non-patent document 11) and that the phenomenon that the neutrophils form the NETs and are put to death, namely, NETosis is a different process from apoptosis or necrosis (Non-patent document 10).

There is prior art of preventing an autoimmune disease such as type I diabetes or rheumatoid arthritis by use of milk-derived basic protein fragment as an active ingredient (Patent document 1). This prior art is specialized for adjustment of immunocyte, mainly, lymphocyte, suppression of inflammatory cytokine, and the like, but is not regarding the action of inhibiting the formation of the NETs.

So far, no therapeutic drug for a disease caused by the formation of the NETs has been reported.

### CITATION LIST

### PATENT LITERATURE

Patent Document 1: Japanese Laid-Open Patent Publication No. 2008-189637

### NON-PATENT LITERATURE

Non-patent Document 1: Volker Brinkmann et al., 2004, Science, 303:1532-1535
Non-patent Document 2: Q Remijsen et al., 2011, Cell Death and Differentiation, 18: 581-588
Non-patent Document 3: Maren von Koeckritz-Blickwede et al., 2008, Blood, 111:3070-3080
Non-patent Document 4: John D. Lippolis et al., 2006, Veterinary Immunology and Immunopathology, 113:248-255
Non-patent Document 5: Fuchs TA et al., 2010, Proc Natl Acad Sci USA, 107:15880-15885
Non-patent Document 6: Garcia-Romo GS et al., 2011 Sci Transl Med, 3:73ra20
Non-patent Document 7: Lande R et al., 2011 Sci Transl Med, 3:73ra19
Non-patent Document 8: Kessenbrock K et al., 2009, Nat Med, 6:623-625
Non-patent Document 9: Bosch X, 2009, J Am Soc Nephrol, 8:1654-1656
Non-patent Document 10: Tobias A. Fuchs et al., 2007, J Cell Biol, 176:231-241
Non-patent Document 11: K. Akong-Moore et al., 2012, PLOS ONE, 7:e42984
Non-patent Document 12: Makoto Naito et al., 2010, Seibutsu Shiryo Bunseki (Analysis of Biological Samples) 33:329-338

### DISCLOSURE OF THE INVENTION

An object of the present invention is to provide a fundamental therapeutic drug for a disease caused by the formation of leukocyte extracellular traps, especially a safe and effective therapeutic drug, and also a therapeutic method, suitable for preservation of remission (suppression of relapse) performed for a long period of time.

As a result of active studies made for the purpose of solving the above-described problem, the present inventors found that lactoferrin exhibits a significant suppressing effect on the formation of NETs and realizes fundamental therapy of NETs-related diseases. It has also been found that the present invention significantly improves the survival rate of model animals for ANCA associated vasculitis, local Shwartzman reaction and disseminated intravascular coagulation (DIC), which diseases are caused by the formation of the NETs.

The present invention is defined in the appended claims.
Also disclosed is the following.
[1] A composition for inhibiting formation of leukocyte extracellular traps, comprising lactoferrin.
[2] The composition according to [1] above, wherein the lactoferrin is in an amount of 0.001 to 10 g/kg/day.
[3] The composition according to [1] above, wherein the lactoferrin is derived from human.
[4] The composition according to [1] above, wherein the lactoferrin is a protein selected from the group consisting of (a) to (c):
   (a) a protein formed of either one of amino acid sequences of SEQ ID NOS: 1 to 5;
   (b) a protein formed of either one of amino acid sequences of SEQ ID NOS: 1 to 5, in which 1 to 66 amino acids are deleted, substituted, inserted and/or added, the protein having activity of inhibiting the formation of the leukocyte extracellular traps; and
   (c) a protein having an amino acid sequence having an amino acid sequence identity of 90% or greater with either one of amino acid sequences of SEQ ID NOS: 1 to 5, the protein having activity of inhibiting the formation of the leukocyte extracellular traps.
[5] The composition according to any one of [1] to [5] above, wherein the leukocytes are one selected from the group consisting of neutrophils, eosinophil granulocytes, basophil granulocytes, monocytes, macrophages, and mast cells.
[6] The composition according to [5] above, wherein the leukocytes are neutrophils.
[7] A composition for treating a disease associated with formation of leukocyte extracellular traps, the composition comprising lactoferrin.
[8] The composition according to [7] above, wherein the lactoferrin is in an amount of 0.001 to 10 g/kg/day.
[9] The composition according to [7] above, wherein the lactoferrin is derived from human.
[10] The composition according to [7] above, wherein the lactoferrin is a protein selected from the group consisting of (a) to (c):
   (a) a protein formed of either one of amino acid sequences of SEQ ID NOS: 1 to 5;
   (b) a protein formed of either one of amino acid sequences of SEQ ID NOS: 1 to 5, in which 1 to 66 amino acids are deleted, substituted, inserted and/or added, the protein having activity of inhibiting the formation of the leukocyte extracellular traps; and
   (c) a protein having an amino acid sequence having an amino acid sequence identity of 90% or greater with either one of amino acid sequences of SEQ ID NOS: 1 to 5, the protein having activity of inhibiting the formation of the leukocyte extracellular traps.
[11] The composition according to any one of [7] to [10] above, wherein the leukocytes are one selected from the group consisting of neutrophils, eosinophil granulocytes, basophil granulocytes, monocytes, macrophages, and mast cells.
[12] The composition according to any one of [7] to [11] above, wherein the disease is one selected from the group consisting of ANCA associated vasculitis, systemic lupus erythematosus, local Shwartzman reaction, acute kidney injury (AKI) accompanied by ischemia reperfusion injury, and disseminated intravascular coagulation.
[13] The composition according to any one of [7] to [12] above, which is in a form of food.
[14] The composition according to any one of [7] to [12] above, which is in a form of an injection agent.
[15] The composition according to any one of [7] to [13] above, which is orally administrable.
[16] A method for inhibiting formation of leukocyte extracellular traps, the method comprising administering lactoferrin to a patient.
[17] A therapeutic method for a disease associated with formation of leukocyte extracellular traps, the therapeutic method comprising administering lactoferrin to a patient.

Disclosed is a therapeutic method with little side effect for a disease caused by the formation of the leukocyte extracellular traps. The method has little side effect and therefore has an advantage of being safely usable for a wide range of patients and people having possibility of becoming patients.

The compositions for inhibition and treatment according to the present disclosure are usable for a wide range of subjects including subjects with immune system depression such as senior people, cancer patients and the like, and subjects who have infectious diseases or complications or who had tuberculosis. The present disclosure provides a therapeutic drug and a therapeutic method for a disease associated with the formation of the leukocyte extracellular traps that have little side effect even if being used for a long period of time. The compositions for inhibition and treatment according to the present disclosure are especially useful as a drug that is usable for a long period of time for treating the above-described diseases, as a drug that suppresses relapse after an acute symptom of a subject remits, or a therapeutic drug for the above-described diseases that become chronic.

### BRIEF DESCRIPTION OF DRAWINGS

FIG. 1-A is a graph showing an ability of bovine lactoferrin of inhibiting the formation of NETs.
FIG. 1-B is a graph showing an ability of human lactoferrin of inhibiting the formation of the NETs.
FIG. 1-C provides fluorescence micrographs showing how human lactoferrin inhibits the formation of the NETs.
FIG. 1-D is a graph showing the effect, provided by human lactoferrin, of suppressing DNAs from being released along with the formation of the NETs.
FIG. 1-E provides electron micrographs showing how human lactoferrin suppresses DNAs from being released along with the formation of the NETs.
FIG. 2 is a graph showing the effect, provided by human lactoferrin, of suppressing DNAs from being released along with the formation of the NETs.
FIG. 3 is a graph showing the effect, provided by oral administration of bovine lactoferrin, of increasing the survival rate of ANCA associated vasculitis model animals.
FIG. 4-A is a graph showing the effect, provided by oral administration of bovine lactoferrin, of decreasing the antibody titer of MPO-ANCA in the blood of the ANCA associated vasculitis model animals.
FIG. 4-B is a graph showing the effect, provided by oral administration of bovine lactoferrin, of decreasing the DNA concentration in the blood of the ANCA associated vasculitis model animals.
FIG. 4-C provides micrographs showing how the disease condition of kidney tissues of the ANCA associated vasculitis model animals are improved by oral administration of bovine lactoferrin.
FIG. 5-A provides photographs showing how subcutaneous bleeding of LSR model animals is improved by oral administration of bovine lactoferrin.
FIG. 5-B is a graph showing, with scores, the improvement in the subcutaneous bleeding of the LSR model animals realized by oral administration of bovine lactoferrin.
FIG. 5-C provides micrographs showing how skin tissues of the LSR model animals are improved by oral administration of bovine lactoferrin.
FIG. 6-A is a graph showing that the DNA concentration in air pouches in the LSR model animals decreases by oral administration of bovine lactoferrin.
FIG. 6-B provides micrographs showing how the release of DNAs in the air pouches in the LSR model animals is suppressed by oral administration of bovine lactoferrin.
FIG. 7 is a graph showing the results of evaluation of the survival rate/lifetime extension after administration of histone.
FIG. 8 is a graph showing the effect of hemostasis on histone-induced thrombus model mice, provided by administration of lactoferrin to the tails thereof.
FIG. 9 provides photographs showing the effect, provided by lactoferrin, of suppressing bleeding in lung tissues of the histone-induced thrombus model mice.
FIG. 10 is a graph showing that the inhibition of the formation of the NETs is activity specific to lactoferrin.

### DESCRIPTION OF EMBODIMENTS

Hereinafter, the present invention will be described in detail.

### 1. Composition for inhibition of formation of leukocyte extracellular traps

In a first embodiment, the present disclosure provides a lactoferrin-containing composition for inhibition of formation of leukocyte extracellular traps (hereinafter, referred to as the "composition for inhibition according to the present disclosure").

Extracellular traps have been reported as being formed by many types of leukocytes. For example, extracellular traps have been reported as being formed by neutrophils (Brinkmann, V., et al., Science 2004; 303:1532-1535), basophil granulocytes (Yousefi, S., et al., Nat Med 2008; 14:949-953), mast cells (von Koeckritz-Blickwede M, et al., Blood 2008; 111:3070-3080), and monocytes (Webster SJ, et al., J Immunol 2010; 185:2968-2979; for example, macrophages (Chow, O.A., et al., Cell Host & Microbe, Volume 8, Issue 5, 445-454, 18 November 2010)), and the like.

The extracellular traps formed by such types of leukocytes have been reported to have a common feature of releasing fiber components mainly containing DNAs and granule proteins (Simon, D., et al, Allergy 68 (2013) 409-416).

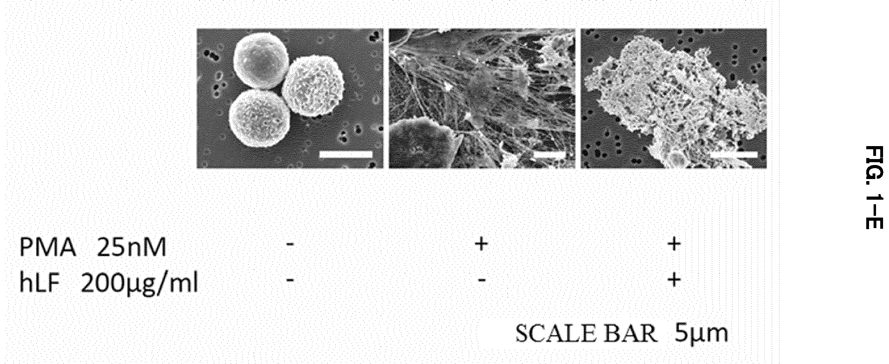

The composition for inhibition according to the present disclosure aggregates and/or condenses the fiber components and thus can suppress the release of the fiber components (see FIG. 1-E). Thus, it is understood that use of the composition for inhibition according to the present disclosure aggregates and/or condenses the fiber components, which would be otherwise released from the neutrophils used in the examples and also the other types of leukocytes (e.g., basophil granulocytes, mast cells, monocytes (e.g., macrophages)) at the time of formation of the extracellular traps, and thus can inhibit the formation of the extracellular traps by these types of leukocytes.

Lactoferrin, which is an active ingredient of the composition for inhibition according to the present disclosure, may be any lactoferrin derived from mammals with no specific limitation. The lactoferrin is preferably derived from mammalian milk that is drinkable by human (e.g., milk of cow, goat, sheep, human), and is more preferably derived from human milk. Alternatively, lactoferrin may be derived from neutrophils of the mammals.

Amino acid sequences of lactoferrin derived from various types of mammals are known (see Table 1 below).

**Table 1**

| Lactoferrin derived from: | Genbank Accession No. | SEQ ID NO |
|---|---|---|
| Human | NP_001186078.1 | 1 |
| Cow | NP_851341.1 | 2 |
| Sheep | ACT76166.1 | 3 |
| Goat | AAA97958.1 | 4 |
| Horse | CAA09407.1 | 5 |

In an embodiment, lactoferrin used for the composition for inhibition according to the present disclosure is a protein selected from the group consisting of (a) to (c) below:
(a) a protein formed of either one of amino acid sequences of SEQ ID NOS: 1 to 5;
(b) a protein formed of either one of amino acid sequences of SEQ ID NOS: 1 to 5, in which 1 to 66 amino acids are deleted, substituted, inserted and/or added, the protein having activity of inhibiting the formation of the leukocyte extracellular traps; and
(c) a protein having an amino acid sequence having an amino acid sequence identity of 90% or greater with either one of amino acid sequences of SEQ ID NOS: 1 to 5, the proteins having activity of inhibiting the formation of the leukocyte extracellular traps.

The protein of (b) or (c) above is typically a variant of either one of polypeptides of SEQ ID NOS: 1 to 5 naturally existent, but encompasses proteins that can be artificially acquired by use of a site-specific mutation induction method described in, for example, "Sambrook & Russell, Molecular Cloning: A Laboratory Manual Vol. 3, Cold Spring Harbor Laboratory Press 2001", "Ausubel, Current Protocols in Molecular Biology, John Wiley & Sons 1987-1997", "Nuc. Acids. Res., 10, 6487 (1982)", "Proc. Natl. Acad. Sci. USA, 79, 6409 (1982)", "Gene, 34, 315 (1985)", "Nuc. Acids. Res., 13, 4431 (1985)", "Proc. Natl. Acad. Sci. USA, 82, 488 (1985)", and the like.

In this specification, the "protein formed of either one of amino acid sequences of SEQ ID NOS: 1 to 5, in which 1 to 66 amino acids are deleted, substituted, inserted and/or added, the protein having activity of inhibiting the formation of the leukocyte extracellular traps" encompasses proteins formed of either one of amino acid sequences of SEQ ID NOS: 1 to 5, in which, for example, 1 to 66 amino acid residues, 1 to 65 amino acid residues, 1 to 60 amino acid residues, 1 to 55 amino acid residues, 1 to 50 amino acid residues, 1 to 49 amino acid residues, 1 to 48 amino acid residues, 1 to 47 amino acid residues, 1 to 46 amino acid residues, 1 to 45 amino acid residues, 1 to 44 amino acids, 1 to 43 amino acid residues, 1 to 42 amino acid residues, 1 to 41 amino acid residues, 1 to 40 amino acid residues, 1 to 39 amino acid residues, 1 to 38 amino acid residues, 1 to 37 amino acid residues, 1 to 36 amino acid residues, 1 to 35 amino acid residues, 1 to 34 amino acid residues, 1 to 33 amino acid residues, 1 to 32 amino acid residues, 1 to 31 amino acid residues, 1 to 30 amino acid residues, 1 to 29 amino acid residues, 1 to 28 amino acid residues, 1 to 27 amino acid residues, 1 to 26 amino acid residues, 1 to 25 amino acid residues, 1 to 24 amino acid residues, 1 to 23 amino acid residues, 1 to 22 amino acid residues, 1 to 21 amino acid residues, 1 to 20 amino acid residues, 1 to 19 amino acid residues, 1 to 18 amino acid residues, 1 to 17 amino acid residues, 1 to 16 amino acid residues, 1 to 15 amino acid residues, 1 to 14 amino acid residues, 1 to 13 amino acid residues, 1 to 12 amino acid residues, 1 to 11 amino acid residues, 1 to 10 amino acid residues, 1 to 9 (1 to several) amino acid residues, 1 to 8 amino acid residues, 1 to 7 amino acid residues, 1 to 6 amino acid residues, 1 to 5 amino acid residues, 1 to 4 amino acid residues, 1 to 3 amino acid residues, 1 to 2 amino acid residues, or 1 amino acid residue is deleted, substituted, inserted and/or added, the proteins having activity of inhibiting the formation of the leukocyte extracellular traps. Generally, the number of the amino acid residues deleted, substituted, inserted and/or added is preferably as small as possible.

Such a protein encompasses proteins having an at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% amino acid sequence identity with either one of amino acid sequences of SEQ ID NOS: 1 to 5, and the proteins having activity of inhibiting the formation of the leukocyte extracellular traps. Generally, the degree of the amino acid sequence identity is preferably as high as possible.

Regarding "activity of inhibiting the formation of the leukocyte extracellular traps", the leukocytes are derived from an organism that forms the leukocyte extracellular traps. The leukocytes are preferably derived from vertebrata, and more preferably derived from mammals. Examples of the mammals include human, cow, horse, goat, sheep, dog, and cat. Preferably, the mammal is human.

Preferably, the leukocytes are derived from the above-listed organism and also are one selected from the group consisting of neutrophils, eosinophil granulocytes, basophil granulocytes, monocytes, macrophages, and mast cells. More preferably, the leukocytes are one selected from the group consisting of neutrophils, basophil granulocytes, monocytes, macrophages, and mast cells. More preferably, the leukocytes are neutrophils.

The leukocytes are cultured in the presence of, or in the absence of, lactoferrin, and the culturing system is observed by a microscope to confirm that the formation of the extracellular traps decreases in the presence of lactoferrin. Thus, it can be confirmed that lactoferrin has activity of inhibiting the formation of the leukocyte extracellular traps can be confirmed.

Preferably, the leukocytes are treated with an extracellular trap formation stimulant (paramethoxyamphetamine (PMA), lipopolysaccharide (LPS), etc.) before lactoferrin is added thereto.

Alternatively, the culture supernatant of the culturing system is recovered, and the DNA concentration in the supernatant is measured. In this manner also, it can be confirmed that lactoferrin has activity of inhibiting the formation of the leukocyte extracellular traps. The DNA concentration is of the DNAs released into the culture supernatant mainly at the time of the formation of the extracellular traps.

In the case where the formation of the extracellular traps is inhibited by lactoferrin, the DNA concentration in the supernatant is lower than that in the absence of lactoferrin.

In this case also, it is preferable that the leukocytes are treated with an extracellular trap formation stimulant (paramethoxyamphetamine (PMA), lipopolysaccharide (LPS), etc.) before lactoferrin is added thereto.

The DNA concentration can be simply measured by use of a commercially available kit (Picogreen dsDNA assay reagent (P11496 Invitrogen)).

For the culturing conditions of each type of leukocytes and the method for confirming whether the extracellular traps have been formed, the following may be referred to: neutrophils (Brinkmann, V., et al., Science 2004; 303:1532-1535), A. K Gupta. FEBS letters 2010; 584:3193-3197, D J Novo. Antimicrob Agents Chemother. 2000; 44(4):827-34), basophil granulocytes (Yousefi, S., et al., Nat Med 2008; 14:949-953), mast cells (von Köckritz-Blickwede M, et al., Blood 2008; 111:3070-3080), monocytes (Webster SJ, et al., JImmunol 2010; 185:2968-2979: for example, macrophages (Chow, O.A., et al., Cell Host & Microbe, Volume 8, Issue 5, 445-454, 18 November 2010)).

The expression provided regarding an amino acid sequence of each protein of the present disclosure that "one or a plurality of (e.g., 2 to 9) amino acid residues are deleted, substituted, inserted and/or added" indicates that one or a plurality of amino acid residues are deleted, substituted, inserted and/or added at any position among one or a plurality of positions of the amino acid sequence. Two or more among deletion, substitution, insertion and addition may occur at the same time.

Examples of mutually substitutable amino acid residues will be shown below. Amino acid residues included in the same group are mutually substitutable. Group A: leucine, isoleucine, norleucine, valine, norvaline, alanine. 2-aminobutyric acid, methionine, o-methylserine, t-butylglycine, t-butylalanine, cyclohexylalanine: group B: aspartic acid, glutamic acid, isoaspartic acid, isoglutamic acid, 2-aminoadipic acid, 2-aminosberic acid; group C: asparagine, glutamine; group D: lysine, arginine, orthinine, 2,4-diaminobutyric acid, 2,3-diaminopropionic acid; group E: proline, 3-hydroxyproline, 4-hydroxyproline; group F: serine, threonine, homoserine; group G: phenylalanine, tyrosine.

The lactoferrin used in the present disclosure may be modified with a compound. For example, the lactoferrin may be a modified protein bonded with polyethyleneglycol (Japanese Patent No. 4195486, Japanese Patent No. 4261531, International Publication WO2009/113743) or a fused protein fused with another protein or a fragment thereof (e.g., protein stable in the blood, i.e., IgG, albumin or a fragment thereof, etc.) (Japanese Patent Application No. 2012-98085).

### 2. Therapeutic method

By use of the composition for inhibition according to the present disclosure, the formation of the leukocyte extracellular traps can be effectively inhibited. Namely, the use of the composition for inhibition according to the present disclosure can provide therapy of a disease associated with the formation of the leukocyte extracellular traps (hereinafter, the therapeutic method using the composition for inhibition according to the present disclosure will be referred to as the "therapeutic method of the present disclosure").

In this specification, the term "therapy" generally indicates improving a symptom of a human or a mammal other than the human. The term "improvement" indicates that the degree of the disease is alleviated or is not deteriorated as compared with in the case where, for example, lactoferrin is not administered. The term "therapy" also encompasses "prevention".

In this case, the target of therapy (patient) is an organism suffering, or having a risk of suffering, from a disease associated with the formation of the leukocyte extracellular traps. The target of therapy is preferably vertebrata, and is more preferably a mammal. The mammal is selected from the group consisting of human, cow, horse, goat, sheep, dog and cat. The target of therapy is still more preferably human.

Regarding the therapeutic method of the present disclosure, the "disease associated with the formation of the leukocyte extracellular traps" may be any disease, with no specific limitation, by which the formation of the leukocyte extracellular traps is observed to increase in the body of the patient.

Such diseases include, for example, ANCA associated vasculitis (Wegener's granulomatosis, microscopic polyangitis, allergic granulomatosis-angitis, etc.), acute kidney injury (AKI) accompanied by ischemia reperfusion injury, systemic lupus erythematosus (SLE), appendicitis, aspergillosis, pneumonia, infection with *Diplococcus pneumoniae,* necrotizing fasciitis, infection with *Streptococus,* sepsis, preeclampsia, Crohn's disease, Schistosomiasis, periodontitis, tuberculosis, mastitis, malaria, cystic fibrosis, and thrombosis diseases such as deep venous thrombosis (von Bruhl, M. L., et al., J Exp Med. 2012 Apr 9; 209(4):819-35), myocardial infarction (de Boer, O. J., Thromb Haemost. 2013 Feb; 109(2):290-7), tumor-related thrombosis (Demers, M., Proc Natl Acad Sci USA; 2012 Aug 7; 109(32):13076-81), disseminated intravascular coagulation (DIC) (Tobias A. et al., blood, 29 September 2011, vol. 118, no. 13, pp. 3708-3714), and the like (Non-patent documents 2 and 12). Vasculitis syndrome is classified into large vessel vasculitis, medium vessel vasculitis, and small vessel vasculitis, depending on the size of the vessel in which the disease occurs. The NETs-related diseases such as ANCA associated vasculitis, SLE and the like mentioned above are classified into the small vessel vasculitis. However, DIC is often developed together with a severe case of polyarteritis nodosa, which is medium vessel vasculitis (Guidelines on diagnosis and therapy of circulatory system diseases (report by the 2006-2007 joint research team)), sepsis or solid cancer. With these diseases, the cytotoxicity caused by the formation of the leukocyte extracellular traps (e.g., NETs) causes vascular endothelial dysfunction, and thus organ dysfunction is caused. With the above-mentioned thrombosis diseases, the formation of the leukocyte extracellular traps (e.g., NETs) acts as a trigger to promote cascade of the formation of thrombus. It is considered that lactoferrin suppresses the formation and release/diffusion of the leukocyte extracellular traps (e.g., NETs) to prevent vascular endothelial dysfunction, also suppresses the cascade of the formation of thrombosis to provide an action of protecting the organs, and thus has a therapeutic effect for the above-mentioned diseases.

The diseases which are targets of therapeutic method of the present disclosure are preferably ANCA associated vasculitis (Wegener's granulomatosis, microscopic polyangitis, allergic granulomatosis-angitis, etc.), systemic lupus erythematosus, local Shwartzman reaction, and acute kidney injury (AKI) accompanied by ischemia reperfusion injury; and are more preferably microscopic polyangitis accompanied by increase in the antibody titer of MPO-ANCA (myeloperoxidase specific anti-neutrophil cytoplasmic antibody) in the blood, allergic granulomatosis-angitis, and disseminated intravascular coagulation (DIC).

It has been reported that lactoferrin is used for treating autoimmune diseases such as type I diabetes and rheumatoid arthritis (Patent Document 1). However, the autoimmune diseases such as type I diabetes and rheumatoid arthritis are not considered to be caused mainly by the formation of the leukocyte extracellular traps. Therefore, it is highly possible that lactoferrin does not act via the formation of the extracellular traps in the therapy described in the above-described report.

Namely, according to the present disclosure, lactoferrin exhibits a therapeutic effect on the above-mentioned diseases by a completely novel mechanism, more specifically, a mechanism of inhibiting the formation of the leukocyte extracellular traps.

Therapy of the diseases caused by SLE or ANCA uses steroid, an immunosuppressing drug or the like. Such a therapeutic method has problems of imposing a physical load (side effect or the like) on the patient, causing the patient to suffer, and having a high risk of inducing another disease (infectious disease).

By contrast, according to the present disclosure, lactoferrin contained in food is used as an active ingredient. This is advantageous in causing fewer side effects, not causing the patient to suffer, and having a lower risk of inducing another disease.

### 3. Composition

In another embodiment, the present disclosure provides a lactoferrin-containing composition for treating a disease associated with the formation of the leukocyte extracellular traps (hereinafter, referred to as the "composition of the present disclosure").

The term "composition" indicates a composition containing an additive such as a carrier or the like used in preparation of an active ingredient useful in the present disclosure (lactoferrin, etc.).

Regarding the composition of the present disclosure, the "lactoferrin" and the "disease associated with the formation of the leukocyte extracellular traps" are as described above.

The administration route of the composition of the present disclosure may be any of generally used routes with no specific limitation. Specific examples of the administration route include oral administration, sublingual administration, transnasal administration, pulmonary administration, administration via alimentary canal, transdermal administration, instillation, intravenous injection, subcutaneous injection, intramuscular injection, intraperitoneal injection, local injection, and surgical implant. Preferable examples of the administration route are oral administration and intravenous injection.

The composition of the present disclosure may be provided as a solid formulation such as capsule, tablet, powder or the like; a liquid formulation such as solution, suspension, emulsion or the like; or a semi-liquid formulation such as ointment, cream, paste or the like.

In the case of being orally administered, the composition is preferably provided as a solid formation. In the case of being injected, the composition is preferably provided as a solid formulation encompassing a formulation realized by lyophilization or a liquid formulation.

In the case of being orally administered, the composition of the present disclosure is more preferably prepared as an enteric formulation. Lactoferrin orally taken is known to be easily digested with pepsin in the stomach. The composition prepared as an enteric formulation is taken into the body at a higher rate (Takeuchi et al., Exp Physiol. 2006 Nov; 91(6):1033-40). In this case, it is desirable that lactoferrin powder is compressed in a dry state and coated with an enteric coating material because lactoferrin is thermally unstable when containing moisture (Japanese patents regarding NRL formulation: Japanese Patent No. 4050784 regarding granules, Japanese Patent No. 4592041 regarding tablets).

Lactoferrin may be taken by human or an animal other than the human as food or feed as being added to the food or feed. A method for producing such food or feed is known to a person of ordinary skill in the art. Lactoferrin may also be formulated as a solution formation, more specifically, as an injection agent.

Alternatively, such lactoferrin or lactoferrin degradation may be added to a nutrient, food, drink or the like as it is or in the form of a formulation.

Lactoferrin may be used independently or in a combination with another pharmacologically acceptable component.

For example, a composition as a formulation for oral administration such as powder, granule, tablet, capsule or the like is prepared by a normal method by use of starch, lactose, white sugar, mannitol, carboxymethycellulose, corn starch, inorganic salt or the like. For this type of composition, other than the excipients as listed above, a coating agent, a binder, a disintegrant, a surfactant, a lubricant, a fluidity enhancer, a colorant, a flavor material or the like is usable when necessary.

The composition of the present disclosure may contain lactoferrin, which is an active ingredient, in a therapeutically effective amount. The expression "therapeutically effective amount" refers to an amount of lactoferrin as an active agent which, when being administered to the target, alleviates or does not deteriorate the symptom of the disease associated with the formation of the leukocyte extracellular traps as compared with in the case where lactoferrin is not administered. The therapeutically effective amount" encompasses an amount that is effective for prevention.

In the case where, for example, the composition may be orally administered, the therapeutically effective amount is 0.001 to 10 g/kg/day, 0.005 to 10 g/kg/day, 0.01 to 10 g/kg/day, or 0.01 to 5 g/kg/day. In the case where the composition is administered to human, the therapeutically effective amount is generally 10 mg to 15,000 mg, 10 mg to 12,000 mg, 10 mg to 10,000 mg, 20 mg to 10,000 mg, 20 mg to 8,000 mg, 30 mg to 8,000 mg, or 30 mg to 6,000 mg per day. Such a dose per day may be administered at once or as being divided into several times to a patient who needs therapy of a disease associated with the formation of the leukocyte extracellular traps.

The dose and frequency of administration of the composition of the present disclosure vary in accordance with various factors including the species, body weight, gender, age, and degree of advancement of the tumor disease of the target, and the administration route to the target. A person of ordinary skill in the art such as a physician, a veterinarian, a dentist, a pharmacist or the like could determine the dose of administration in consideration of these factors.

In the above, typical values are listed regarding the therapeutically effective amount, and the dose and frequency of administration. Even a value above or below the list may sufficiently be therapeutically effective. Therefore, even a value above or below the therapeutically effective amount, or the dose or frequency of administration is encompassed in the therapeutically effective amount, or the dose or frequency of administration of the composition of the present disclosure.

### EXAMPLES

Hereinafter, the present disclosure will be described in detail by way of examples. The present disclosure is not to be limited to any of the embodiments described in the examples.

### [Example 1] The inhibitory effect of Net formation stimulation with healthy volunteer's peripheral blood by pre-treated lactoferrin

### 1. Method of isolating human neutrophils

From heathy persons, 15 ml to 20 ml of peripheral blood was sampled for one cycle of experiment with an EDTA-containing syringe (needle: 18 to 22 G). After the blood sampling, 3 ml of mono-poly resolving medium (Cat No. DSBN100, DS Pharma Biomedical Co., Ltd.) contained in 15ml conical tube, and then 3.5 ml of the whole blood were gently stacked in the lower-layer mono-poly resolving medium. The 15 ml conical tube was centrifuged at room temperature (15 to 30°C) at 400 × g for 20 minutes by a swing-type centrifuge, and then the conical tube was taken out gently. A brown plasma layer in the top layer and a lymphocyte/monocyte layer immediately below the brown plasma layer were removed by an aspirator or the like. A transparent layer below the lymphocyte/monocyte layer was removed as much as possible. A pale pink layer below the transparent layer was taken out by a Pasteur pipette. In order to wash neutrophils, the pale pink layer was transferred to a test tube containing phosphate-buffered saline (PBS(-): 137 mM sodium chloride, 2.7 mM potassium chloride, 8.1 mM disodium hydrogenphosphate dodecahydrate, 1.47 mM potassium dihydrogenphosphate). The test tube containing the neutrophils was centrifuged at room temperature (15 to 30°C) at 200 × g for 10 minutes. The test tube was taken out, and the supernatant was discarded. 4°C sterilized water was added to the neutrophils in the test tube and left still on ice for 30 seconds to cause hemolysis of erythrocytes mixed therein. After the hemolysis, 45 ml of PBS(-) was added to the test tube, and the test tube was centrifuged at 200 × g for 10 minutes. The test tube was taken out, and the supernatant was discarded. The precipitation was suspended in culture DMEM + 2% human serum Alb (serum human albumin; Product No. A9080 Sigma + 4 mM L-glutamine), and left at 8°C until immediately before being used. The neutrophils were separated in the number of 1 × 10⁶/ml to 1 × 10⁷/ml. The purity, which was obtained by visually counting the post-cytospin sample stained with Giemsa, was 95 to 98%.

### 2. Method of pre-treating the neutrophils (inhibiting the formation of the NETs) and method of inducing NETosis (forming the NETs)

About 1 hour after the isolation of the neutrophils described in "1. Method of isolating human neutrophils" in Example 1, the neutrophils were pre-treated. On an 8 well-µ slide (Cat. No. ib 80826 Ibidi (registered trademark)), the neutrophils were seeded in 400 µl of culture DMEM + 2% human serum Alb (serum human albumin; Product No. A9080 Sigma + 4 mM L-glutamine) at a rate of about 1.0 × 10⁵ cells/well. Drugs (i) to (v) were each added, and pre-treatment was performed at room temperature for 30 minutes: (i) bovine lactoferrin (Product No. 123-04124 Lot: KWG6332 WAKO (registered trademark)) (stock: 100 mg/ml) diluted at 1/500, 1/5000 or 1/50000 to have final concentrations of 200 µg/ml, 20 µg/ml or 2 µg/ml; (ii) human lactoferrin (stock: 100 mg/ml, 200 µl; Cat No. SIGL6793, SIGMA) diluted at 1/500, 1/5000 or 1/50000 to have final concentrations of 200 µg/ml, 20 µg/ml or 2 µg/ml; (iii) DPI (stock: 25 mM; Product No. D2926 Sigma; attached document; solvent: DMSO) diluted at 1/2500 to have a final concentration of 10 µM; (iv) deferoxamine (Product No. D9533 Sigma) (stock: 200 mM) diluted at 1/100 to have a final concentration of 2 mM; (v) trientine (triethylenetetramine dihydrochloride (Sigma Product No. T5033; Lot: 1354380V; stock 100 mM, 100 µl)) (final concentration: 125 µM, 12.5 µM, 1.25 µM)). The pre-treatment was each performed by use of reagent at each concentration. After the pre-treatment, the neutrophils were stimulated with PMA (final concentration: 25 nM, stock: 10 mM in DMSO; Sigma Prod No. P8139).

### 3. Method and results of observation of NETosis caused along with the formation of the NETs

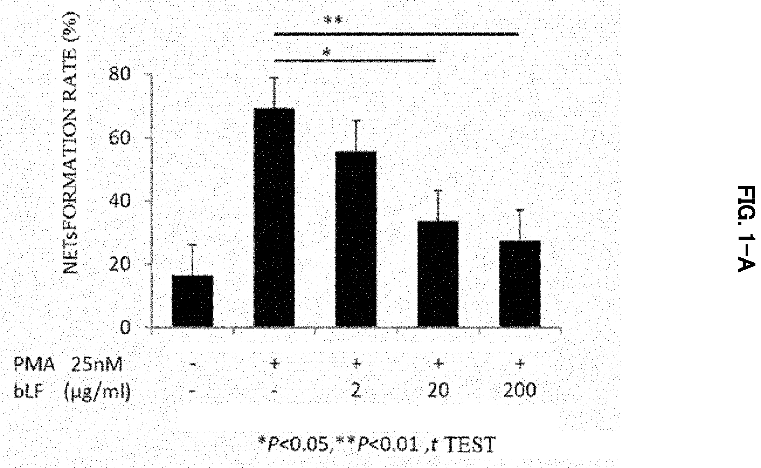

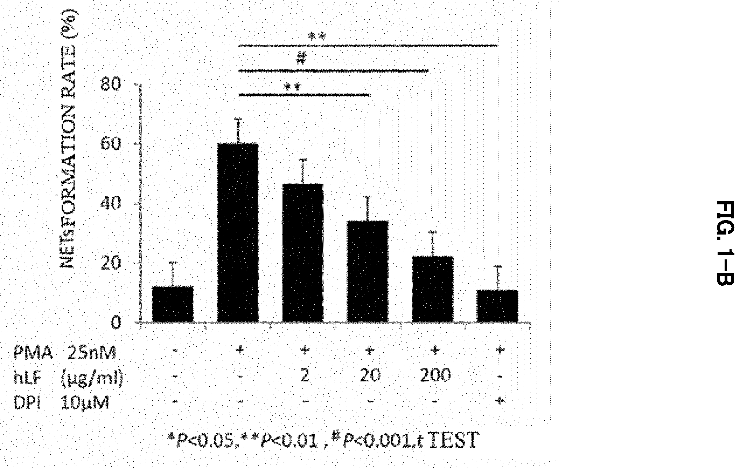

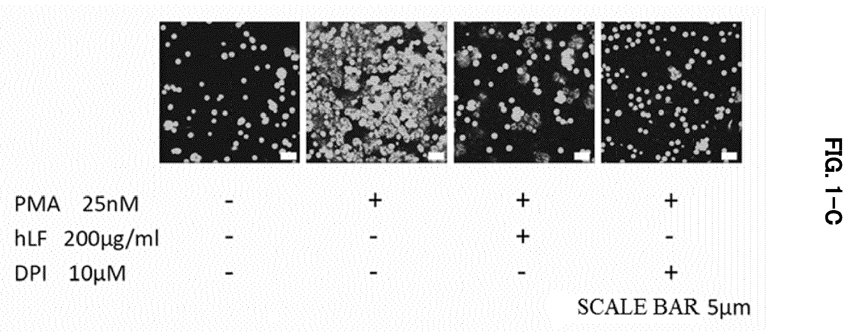

For observation of the formation of the NETs, a confocal microscope (Leica DMI 6000B, Leica (registered trademark)) was used. The number of the neutrophils was visually countered via the microscope (in all the experiments, probe of HySOₓ (final concentration: 500 nM; stock: 10 mM; provided by Professor Yasuteru URANO, Bioinformatics, Biomedical Engineering, Department of Biophysical Medicine, Graduate School of Medicine, The University of Tokyo) (Kenmoku, S., et al., 2007. J Am Chem Soc 129:7313-7318; Setsukinai, K., et al., 2003. J Biol Chem 278:3170-3175) was used, and red fluorescent cells obtained by use of the probe were counted). For the quantization of the NETs, the number of mesh-like structures stained with Sytoxgreen (final concentration: 500 nM; stock: 5 mM, 5µl; Product No. S7020) and TO-PRO-3 (final concentration: 1 µM; stock: 1 mM; Cat No. T3605 Invitrogen (registered trademark)) and released to the outside of the neutrophils was visually counted, referring to A. K Gupta. FEBS letters 2010; 584:3193-3197, D J Novo. Antimicrob Agents Chemother. 2000; 44(4):827-34 [FIG. 1-C]. After the reagent was injected, in order to allow the living cells to be observed at 37°C for a long time by supplying oxygen for drying, 100 µl of silicone oil (AR200 Lot: BCBF0602V ALDRICH Chemistry (registered trademark) 85419) was injected to form an oil layer. The observation time was set to 1 to 8 hours (4 hours in the case where the time duration was not counted until an equilibrium state of the formation of the NETs was obtained), and the samples under observation were kept at 37°C by a heat holding box provided with a microscope. The number of cells which were observed to release the NETs was divided by the total number of cells in one microscopic field, and the obtained value was represented as the rate of the formation of the NETs. In the case where the neutrophils were stimulated only with PMA, the formation of the NETs reached a peak within 2 to 3 hours, and reached a plateau at the fourth hour or thereafter. The formation of the NETs reached a peak within 4 to 5 hours, and reached a plateau at the sixth hour or thereafter because the life of the neutrophils in the blood was 10 to 12 hours. In the case where the neutrophils were not stimulated, the number of the NETs formed was as small as 1/8 to 1/7 of the number of the NETs formed in the case where the neutrophils were stimulated with PMA. In the case where each of the processes described in "2. Method of pre-treating the neutrophils (inhibiting the formation of the NETs) and method of inducing NETosis (forming the NETs)" was performed before the neutrophils were stimulated with PMA, the rate of the NETs formed at the time when the formation of the NETs reached the plateau was as follows as compared with in the case where the neutrophils were stimulated merely with PMA. In the case where the process was performed with bovine lactoferrin or human neutrophil-derived lactoferrin (SIGMA Cat No. SIGL6793) at a concentration of 2 µg/ml or 20 µg/ml, the rate of the NETs formed was half. In the case where the process was performed with bovine lactoferrin or human neutrophil-derived lactoferrin (SIGMA Cat No. SIGL6793) at a concentration of 200 µg/ml, the formation of the NETs was suppressed to 1/4 [FIG. 1-A and FIG. 1-B]. In the case where the process was performed with DPI, the formation of the NETs was suppressed to 1/3. In the case where the process was performed with deferoxamine or trientine, the formation of the NETs was not suppressed.

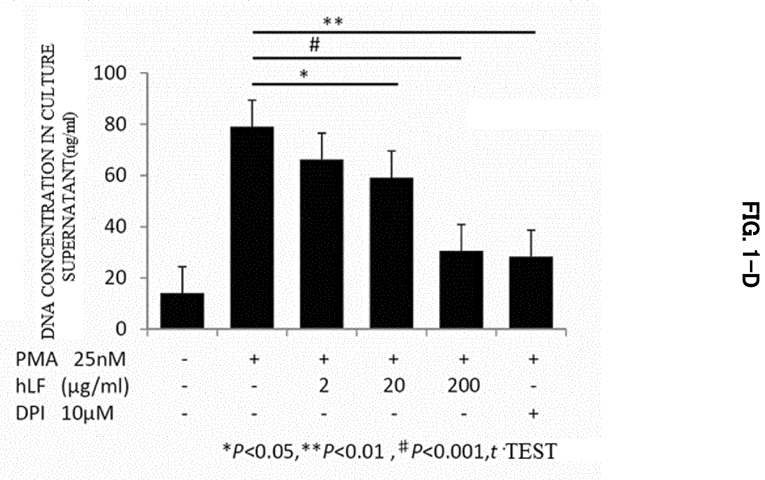

The observation by a scanning electron microscope was performed as follows. Eosinophil granulocytes were seeded on a glass cover or a glass bottom dish for cell culture in the number of 1 × 10⁶ and pre-treated with 200 µg/ml of human lactoferrin or were not pre-treated before stimulation for NETs. Three hours later, the sample was pre-immobilized with 0.1 M phosphoric acid buffer (pH: 7.4) containing 2% glutaraldehyde at 4°C for 1 hour. Next, the sample was post-immobilized with 0.1 M phosphoric acid buffer (pH: 7.4) containing 1% osmium tetroxide at 4°C for 1 hour. After the immobilization, the sample was washed with 60% ethanol, with 70% ethanol, with 80% ethanol and with 95% ethanol while being gently shaken for 5 to 10 minutes, and immersed twice in 100% ethanol for 5 to 10 minutes to be dehydrated. Critical point drying was performed by substitution with isoamyl acetate for 10 to 15 minutes. The sample were covered with a layer of sublimated osmium tetroxide by use of an osmium plasma coating device (OPC80N, Filgen, Inc.) and observed by a scanning electron microscope (JSM-6320F, JEOL, Ltd.). In the case where the neutrophils were not stimulated, the neutrophils were kept spherical and no extracellular fiber was recognized. In the case where the neutrophils were stimulated with PMA, the cells were broken and many fiber components were formed. By contrast, the neutrophils pre-treated with lactoferrin were observed to form such fiber components in bundles. This indicates that the fibers of the NETs were condensed by lactoferrin [FIG. 1-D].

### 4. Method of measuring the DNA concentration in the culture supernatant

The culture supernatant was recovered and centrifuged at 200 × g for 10 minutes, and the supernatant was transferred to a new microscopic centrifuge tube. The DNA amount was measured by use of Picogreen dsDNA assay reagent (PI 1496 Invitrogen) in accordance with an accompanying protocol. In the case where pre-treatment was performed with human lactoferrin, the DNA concentration in the culture supernatant decreased in a concentration-dependent manner, and thus the release of the DNAs by the NETs was suppressed [FIG. 1-D].

### [Example 2] Inhibitory effect of lactoferrin after the stimulation of the neutrophils to form the NETs in peripheral blood of heathy volunteer

### 1. Method of isolating human neutrophils

From heathy volunteer, 15 ml to 20 ml of peripheral blood was sampled for one cycle of experiment with an EDTA-containing syringe (needle: 18 to 22 G). After the blood sampling, 3 ml of mono-poly resolving medium (Cat No. DSBN100, DS Pharma Biomedical Co., Ltd.) contained in 15ml conical tube, and then 3.5 ml of the whole blood were gently stacked in the lower-layer mono-poly resolving medium. The 15 ml conical tube was centrifuged at room temperature (15 to 30°C) at 400 × g for 20 minutes by a swing-type centrifuge, and then the conical tube was taken out gently. A brown plasma layer in the top layer and a lymphocyte/monocyte layer immediately below the brown plasma layer were removed by an aspirator or the like. A transparent layer below the lymphocyte/monocyte layer was removed as much as possible. A pale pink layer below the transparent layer was taken out by a Pasteur pipette. In order to wash the neutrophils, the pale pink layer was transferred to a test tube containing phosphate-buffered saline (PBS(-): 137 mM sodium chloride, 2.7 mM potassium chloride, 8.1 mM disodium hydrogenphosphate dodecahydrate, 1.47 mM potassium dihydrogenphosphate). The test tube containing the neutrophils was centrifuged at room temperature (15 to 30°C) at 200 × g for 10 minutes. The test tube was taken out, and the supernatant was discarded. 4°C sterilized water was added to the neutrophils in the test tube and left still on ice for 30 seconds to cause hemolysis of erythrocytes mixed therein. After the hemolysis, 45 ml of PBS(-) was added to the test tube, and the test tube was centrifuged at 200 × g for 10 minutes. The test tube was taken out, and the supernatant was discarded. The precipitation was suspended in culture DMEM + 2% human serum Alb (serum human albumin; Product No. A9080 Sigma + 4 mM L-glutamine), and left at 8°C until immediately before being used. The neutrophils were separated in the number of 1 × 10⁶/ml to 1 × 10⁷/ml. The purity, which was obtained by visually counting the post-cytospin sample stained with Giemsa, was 95 to 98%.

### 2. Induction of NETosis of the neutrophils (formation of the NETs) and method of inhibiting the same

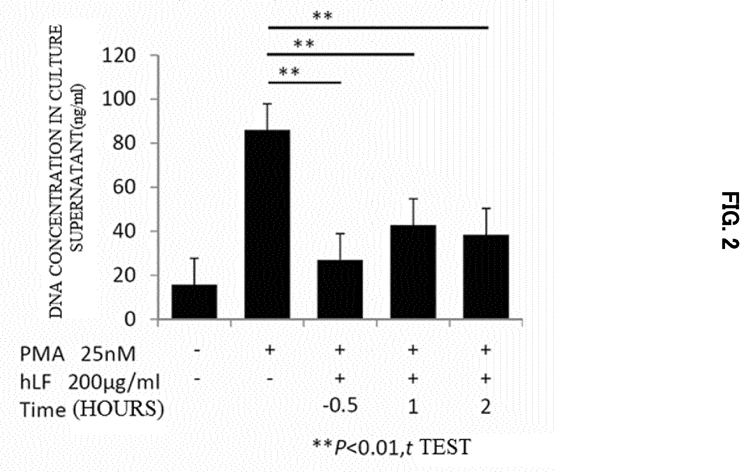

About 1 hour after the isolation of the neutrophils described in "1. Method of isolating human neutrophils" in Example 2, the neutrophils were pre-treated. On an 8-well microslide (Cat. No. ib 80826 Ibidi (registered trademark)), the neutrophils were seeded in 400 µl of culture DMEM + 2% human serum Alb (serum human albumin; Product No. A9080 Sigma + 4 mM L-glutamine) at a rate of about 1.0 × 10⁵ cells/well, and stimulated with PMA (final concentration: 25 nM, stock: 10 mM in DMSO, Sigma Prod No. P8139). At thirty minutes before the stimulation, and at every one hour or every two hours after the stimulation, 200 µg/ml of human lactoferrin (stock: 100 mg/ml, 200 µl; Cat. No. SIGL6793, SIGMA) was added to perform inhibition [FIG. 2].

### 4. Method of measuring the DNA concentration in the culture supernatant

The culture supernatant was recovered and centrifuged at 200 × g for 10 minutes, and the supernatant was transferred to a new microscopic centrifuge tube. The DNA amount was measured by use of Picogreen dsDNA assay reagent (PI 1496 Invitrogen) in accordance with an accompanying protocol. Treatment with human lactoferrin at one hour and two hours after the stimulation was a similar effect to the prior pre-treatment, the DNA concentration in the culture supernatant decreased, resulting in suppression of the release of the DNAs by the NETs [FIG. 2].

### [Example 3] Improvement effect on the survival rate/lifetime extension of ANCA associated vasculitis model SCG/Kj mice (autoimmune disease model animals) by oral administration of lactoferrin

### 1. Production of LF-containing mouse feed and method of feeding the same

Production of standard feed and lactoferrin-containing feed was outsourced to Oriental Kobo Kabushiki Kaisha.

The standard feed was produced as follows. The standard refined feed AIN-93M for nutrition research for mice and rats that was published in 1993 by American Institute of Nutrition (14% of casein, 0.18% of L-cystine, 46.5692% of corn starch, 15.5% of α-corn starch, 10.0% of sucrose, 4.0% of soybean oil, 5.0% of cellulose powder, 3.5% of AIN-93M mineral mixture, 1.0% of AIN-93 vitamin mixture, 0.25% of choline tartrate, 0.0008% of tert-butylhydroquinone) was solidified by a pelleter. The mice were allowed to take the feed freely. The lactoferrin-containing feed was produced as follows. Bovine lactoferrin was mixed in AIN-93M so as to have a final concentration of 2%, and the mixture was solidified by a pelleter. The mice were allowed to take the feed freely.

### 2. Improvement effect on the survival rate/lifetime extension of SCG/Kj (Spontaneous Crescentic Glomerulonephritis-forming mouse/Kinjoh)

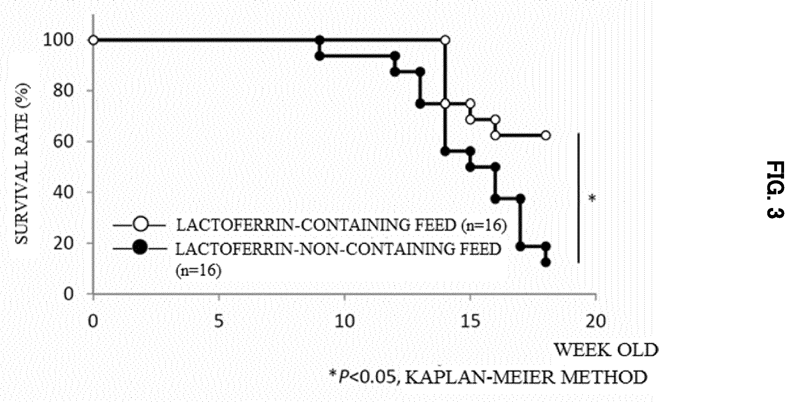

Six to seven week old female SCG/Kj mice having crescentic glomerulonephritis and ANCA associated vasculitis were purchased from BioResource Center, Tsukuba Institute, RIKEN, and allowed to be accustomed for 1 to 2 weeks before being used. The mice were divided into two groups. From the eighth week of age, one group of mice were fed with the lactoferrin-containing feed (n = 16), and the other group of mice were fed with the standard feed (n = 16). The survival rate/lifetime extension was evaluated by use of the Kaplan-Meier method. The group of mice fed with the standard feed gradually started to die from the ninth week of age. At the 18th week of age, two mice were alive. The group of mice fed with the lactoferrin-containing feed started to die from the 14th week of age, and 11 mice were alive at the 18th week of age, demonstrating a significant improvement effect on the survival rate/lifetime extension (statistically significant difference p = 0.0111) [FIG. 3].

### [Example 4] Influence of administration of lactoferrin on the antibody titer of the MPO-ANCA (myeloperoxidase specific anti-neutrophil cytoplasmic antibody) in the blood and on the DNA in the blood of ANCA associated vasculitis model SCG/Kj mice (autoimmune disease model animals)

### 1. Production of LF-containing mouse feed and method of feeding the same

Production of standard feed and lactoferrin-containing feed was outsourced to Oriental Kobo Kabushiki Kaisha.

The standard feed was produced as follows. The standard refined feed AIN-93M for nutrition research for mice and rats that was published in 1993 by American Institute of Nutrition (14% of casein, 0.18% of L-cystine, 46.5692% of corn starch, 15.5% of α-corn starch, 10.0% of sucrose, 4.0% of soybean oil, 5.0% of cellulose powder, 3.5% of AIN-93M mineral mixture, 1.0% of AIN-93 vitamin mixture, 0.25% of choline tartrate, 0.0008% of tert-butylhydroquinone) was solidified by a pelleter. The mice were allowed to take the feed freely. The lactoferrin-containing feed was produced as follows. Bovine lactoferrin was mixed in AIN-93M so as to have a final concentration of 2%, and the mixture was solidified by a pelleter. The mice were allowed to take the feed freely.

### 2. Effective experiment of the MPO-ANCA antibody titer and the DNA in the blood of an ANCA-associated vasculitis model SCG/Kj mice

At least 6 week old female SCG/Kj mice were purchased from BioResource Center, Tsukuba Institute, RIKEN, and used from the eighth week of age. The mice were divided into two groups. One group of mice were fed with the lactoferrin-containing feed (n = 8), and the other group of mice were fed with the standard feed (n = 8). SCG/Kj mice of 8 to 17 weeks of age were caused to inhale diethylether to be anesthetized, and blood was sampled with heparin. The sampled blood was transferred to a 1.5 ml microscopic centrifuge tube, and centrifuged at 4°C at 1000 g for 10 minutes. The supernatant was recovered to be used as plasma.

### 3. Method of measuring antibody titer of the MPO-ANCA in the blood

The measurement of the antibody titer of the MPO-ANCA was performed by use of ELISA (Ishida-Okawara, A., et al, Nephrol Dial Transplant 2004; 19:1708-1715) provided by Prof. Kazuo SUZUKI, formerly in Department of Immunology and Inflammation Control, Graduate School of Medicine, Chiba University. The recombinant mouse MPO was seeded at a certain concentration to a 96-well ELISA plate (TOYOSHIMA) and left at 4°C for 16 hours. After the MPO was left in this manner, the supernatant was discarded, and 300 to 400 µl of PBS(-) was put into each well for washing (the operation was performed 2 to 3 times). After the washing, 300 to 400 µl of 1% PBS(-) solution of bovine serum albumin was put into each well, left at room temperature for 2 hours, and then 300 to 400 µl of PBS(-) was put into each well for washing (the operation was performed 3 to 4 times). After the wells were left in this manner, mouse serum diluted 50-fold with PBS(-) was put into each well and reacted at room temperature for 90 minutes. After the reaction, 300 to 400 µl of PBS(-) was put into each well for washing (the operation was performed 3 to 4 times).

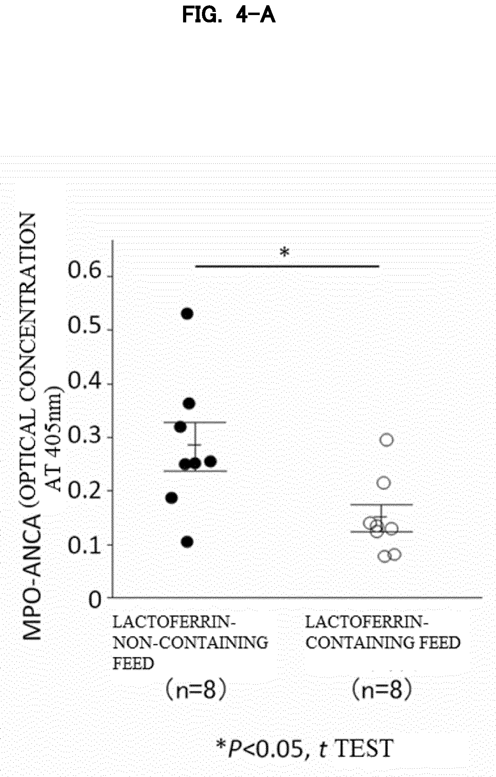

After the washing, anti-mouse IgG antibody labeled with alkaline phosphatase diluted 1000-fold with PBS(-) was put into each well and reacted at room temperature for 2 hours. After the reaction, 300 to 400 µl of PBS(-) was put into each well for washing (the operation was performed 2 to 3 times). 150 µl of 1 mg/ml para-nitrophenylphosphoric acid diluted with PBS(-) was put into each well and reacted for 15 to 30 minutes. An equivalent amount of 0.75 M sodium hydroxide aqueous solution was put into each well to stop the reaction. The measurement was performed at a wavelength of 405 nm. The results of the measurement performed by use of an absorptiometer were that the average antibody titer of the MPO-ANCA of the group of mice fed with the standard feed was 0.283875 and the average antibody titer of the MPO-ANCA of the group of mice fed with the lactoferrin-containing feed was 0.15625. The antibody titer of the MPO-ANCA was recognized to be lower with the group of mice fed with the lactoferrin-containing feed. Thus, the therapeutic effect was recognized (statistically significant difference p = 0.0221) [FIG. 4-A].

### 4. Method of measuring the DNA concentration in the plasma

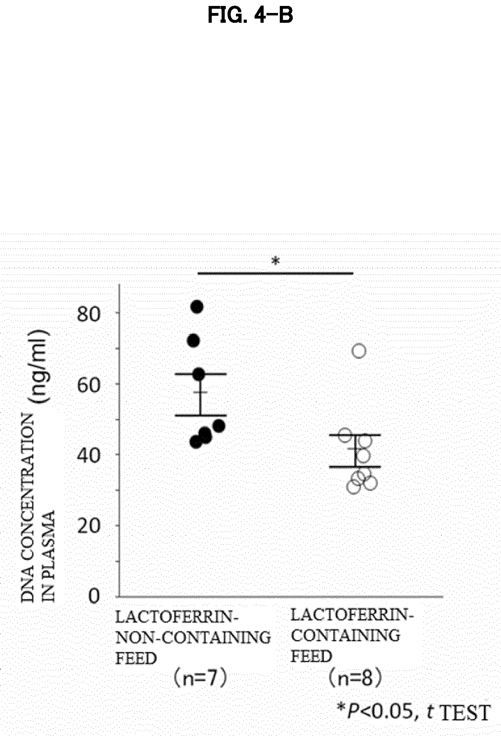

The DNA amount in the plasma was measured by use of Picogreen dsDNA assay reagent (P11496 Invitrogen) in accordance with an accompanying protocol. The group of mice fed with the lactoferrin-containing feed had a smaller DNA amount in the blood than the group of mice fed with the lactoferrin-non-containing feed. This indicates that the release of the DNAs by the NETs was significantly suppressed with the group of mice fed with the lactoferrin-containing feed (p = 0.046) [FIG. 4-B].

### 5. Method of evaluating tissues

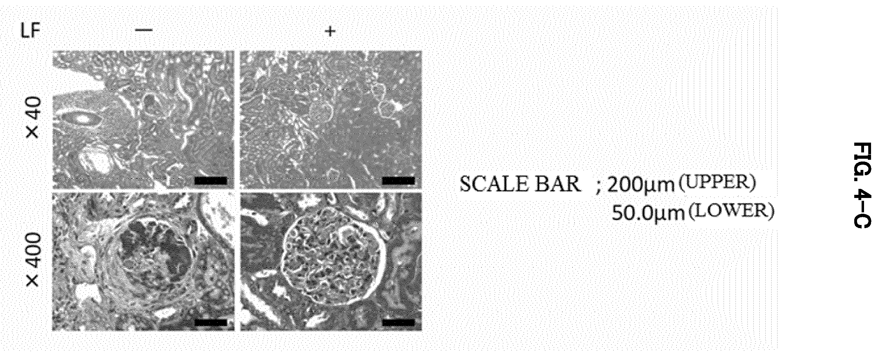

The SCG/Kj mice were put to euthanasia by cervical dislocation, and kidney samples were collected by celiotomy. A 40% paraffin block was created, and tissue sections were formed. Masson trichrome staining was performed, and subcutaneous bleeding was histologically evaluated. The tissue sections were osmosed with 10% formalin solution for 24 hours or longer to be immobilized. Formalin was washed with tap water for 1 hour or longer. The immobilized tissue sections were immersed in 60% ethanol for 1 hour, in 70% ethanol for 1 hour, in 80% ethanol for 1 hour, in 95% ethanol for 1 hour, in 100% ethanol for 1 hour 3 times, in xylene for 1 hour twice, and in paraffin (kept at 65°C) for 1 hour 3 times. Then, the tissue block was created with an embedding tray. The tissue block was cut by a microtome into tissue sections each having a thickness of 20 to 50 nm. The tissue sections were put onto a glass slide and deparaffinized. The deparaffinization was performed as follows. The tissue sections that were completely dry on the glass slide were lightly washed with xylene for 5 minutes 3 times, with 100% ethanol for 1 minute twice, and with 95% ethanol. Then, similarly, the tissue sections were lightly washed with 80% ethanol, with 70% ethanol, with 60% ethanol and with tap water in this order. Then, the tissue sections were immersed in ion exchange water, and then were treated with the Masson trichrome staining. Then, the tissue sections were immersed in a mordanting liquid (10% trichloroacetic acid solution, 10% potassium dichromate solution) for 10 to 15 minutes, washed with tap water for 5 minutes, immersed in an iron hematoxylin solution (2 g hematoxylin, 100 ml of 100% ethanol, 0.5 g of ferric nitrate (III)·9H₂O, 100 ml of 25% hydrochloric acid solution) for 5 minutes, and lightly washed with water. A 1% hydrochloric acid in 70% ethanol was used for separation. The tissue sections were washed with water for 10 minutes to remove the color, and immersed in ion exchange water. The tissue sections were immersed in liquid I (90 ml of 1% Biebrich Scarlet, 10 ml of 1% acidic fuchsine, 1 ml of acetic acid) for 2 to 5 minutes, and lightly washed with water. The tissue sections were immersed in liquid II (5 g of phosphomolybdic acid, 5 g of phosphotungstic acid, 200 ml of distilled water) for 30 minutes or longer, lightly washed with water, immersed in liquid III (2.5 g of aniline blue, 2 ml of acetic acid, 100 ml of distilled water) for 5 minutes, and lightly washed with water. The tissue sections were immersed in 1% aqueous acetic acid for 5 minutes, and quickly washed with water. The tissue sections were lightly washed with 60% ethanol, with 70% ethanol, with 80% ethanol and with 95% ethanol, and then immersed in 100% ethanol for 5 minutes 3 times. The tissue sections were immersed in xylene for 5 minutes 3 times, and covered with a cover glass by use of a mounting agent. The glass slide was dried and then observed by a microscope. Separately, similar tissue sections were stained with hematoxylin-eosin. After being deparaffinized, the tissue sections were washed with running tap water for 3 to 5 minutes, and immersed in a Mayer's hematoxylin solution for 5 minutes. The tissue sections were washed with running water at 25 to 37°C for 3 to 5 minutes, and immersed in an eosin solution for 5 minutes. The tissue sections were lightly washed with 60% ethanol, with 70% ethanol, with 80% ethanol and with 95% ethanol, and then immersed in 100% ethanol for 5 minutes 3 times. The tissue sections were immersed in xylene for 5 minutes 3 times, and covered with a cover glass by use of a mounting agent. The glass slide was dried and then observed by a microscope. With both of the staining methods, the kidney of the group of mice fed with the lactoferrin-containing feed was milder regarding interstitial fibrosis, inflammatory cell infiltration (upper) and crescent body formation (lower) of the tissues than the kidney of the group of mice fed with the lactoferrin-non-containing feed [FIG. 4-C].

### [Example 5] Therapeutic effect of lactoferrin on the local Shwartzman reaction (LSR) model mice (non-autoimmune disease model mice)

### 1. Production of LSR model 1 and its evaluation 1

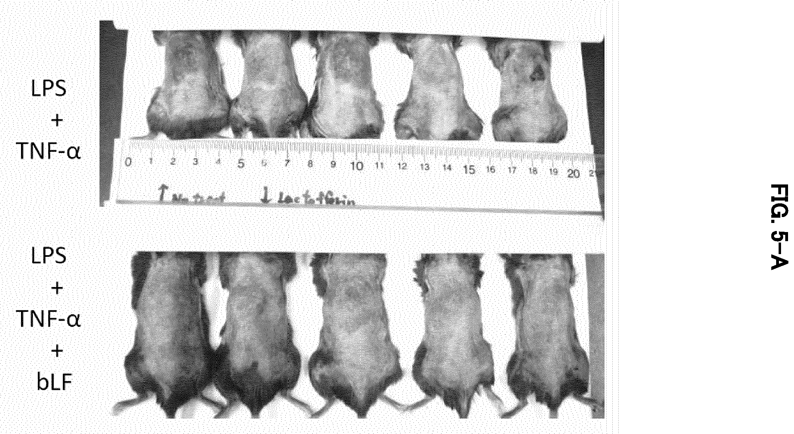

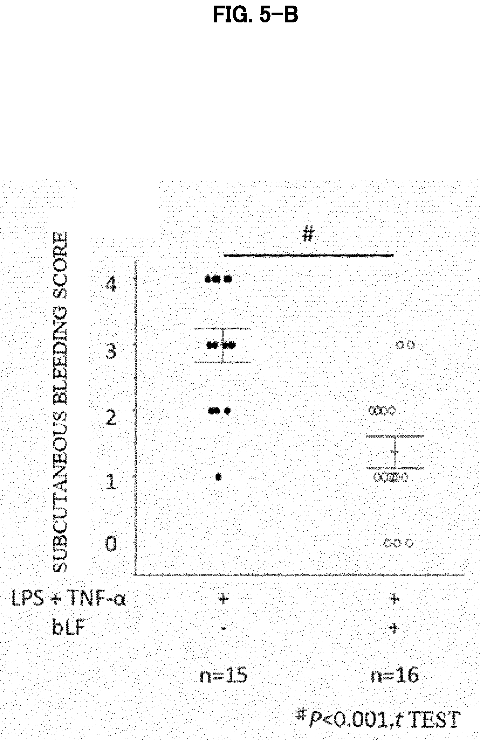

Six week old C57BL/6j mice were allowed to be accustomed for 2 weeks, and from the eighth week of age, fed with the lactoferrin-containing feed or control feed. At the 10th week of age, lipopolysaccharide (LPS; Sigma) derived from Escherichia coli was dissolved in PBS(-) so as to be _ mg/ml. 100 µg of the resultant solution was subcutaneously injected to each mouse by a 30 G needle. The day on which the injection was performed was set as day 1. Mouse recombinant TNF-α (R&D Systems, Inc., Product No. 410-MT) was dissolved in PBS(-) so as to be 100 µg/ml. 0.3 µg of the resultant solution was subcutaneously injected to each mouse at the same site. The day on which the injection was performed was set as day 2. On day 3, the bleeding of the site was evaluated with the naked eye. With the group of mice fed with the lactoferrin-containing feed, the range of subcutaneous bleeding was more significantly suppressed than with the control group of mice fed with the control feed. The severity of the subcutaneous bleeding was observed with the naked eye and numerically evaluated based on the range of bleeding and the state of necrosis as follows: 0: none; 1: mild; 2: moderate; 3: severe; 4: central necrosis [Table 2]. The severity of the group of mice fed with the lactoferrin-containing feed was significantly lower than that of the control group of mice fed with the control feed (p > 0.0001) [FIG. 5-A, B].

**Table 2 Evaluation scores, by naked-eye observation, of the subcutaneous bleeding of the group of mice fed with the lactoferrin-containing feed (n= 16) and the group of mice fed with the lactoferrin-non-containing feed (n= 15)**

| Naked-eye observation | None | Mild | Moderate | Severe | Central necrosis |
|---|---|---|---|---|---|
| Subcutaneous bleeding score | 0 | 1 | 2 | 3 | 4 |

### 2. Method of evaluating the tissues

The mice produced in "1. Production of LSR model 1 and its evaluation" in Example 5 were put to euthanasia by cervical dislocation, and skin samples were taken. A 40% paraffin block was created, and tissue sections were formed. The Masson trichrome staining was performed, and subcutaneous bleeding was histologically evaluated. The tissue sections were osmosed with 10% formalin solution for 24 hours or longer to be immobilized. Formalin was washed away with tap water for 1 hour or longer. The immobilized tissue sections were immersed in 60% ethanol for 1 hour, in 70% ethanol for 1 hour, in 80% ethanol for 1 hour, in 95% ethanol for 1 hour, in 100% ethanol for 1 hour 3 times, in xylene for 1 hour twice, and in paraffin (kept at 65°C) for 1 hour 3 times. Then, the tissue block was created with an embedding tray. The tissue block was cut by a microtome into tissue sections each having a thickness of 20 to 50 nm. The tissue sections were put onto a glass slide and deparaffinized. The deparaffinization was performed as follows. The tissue sections that were completely dry on the glass slide were lightly washed with xylene for 5 minutes 3 times, with 100% ethanol for 1 minute twice, and with 95% ethanol. Then, similarly, the tissue sections were lightly washed with 80% ethanol, with 70% ethanol, with 60% ethanol and with tap water in this order. Then, the tissue sections were immersed in ion exchange water, and then were treated with the Masson trichrome staining. Then, the tissue sections were immersed in a stain fixing solution (10% trichloroacetic acid solution, 10% potassium dichromate solution) for 10 to 15 minutes, washed with tap water for 5 minutes, immersed in an iron hematoxylin solution (2 g of hematoxylin, 100 ml of 100% ethanol, 0.5 g of ferric nitrate (III)·9H₂O, 100 ml of 25% hydrochloric acid solution) for 5 minutes, and lightly washed with water. A solution of 1% hydrochloric acid and 70% ethanol was used for separation. The tissue sections were washed with water for 10 minutes to remove the color, and then immersed in ion exchange water. The tissue sections were immersed in liquid I (90 ml of 1% Biebrich Scarlet, 10 ml of 1% acidic fuchsine, 1 ml of acetic acid) for 2 to 5 minutes, and lightly washed with water. The tissue sections were immersed in liquid II (5 g of phosphomolybdic acid, 5 g of phosphotungstic acid, 200 ml of distilled water) for 30 minutes or longer, lightly washed with water, immersed in liquid III (2.5 g of aniline blue, 2 ml of acetic acid, 100 ml of distilled water) for 5 minutes, and lightly washed with water. The tissue sections were immersed in 1% aqueous acetic acid for 5 minutes, and quickly washed with water. The tissue sections were lightly washed with 60% ethanol, with 70% ethanol, with 80% ethanol and with 95% ethanol, and then immersed in 100% ethanol for 5 minutes 3 times. The tissue sections were immersed in xylene for 5 minutes 3 times, and covered with a cover glass by use of a mounting agent. The glass slide was dried and then observed by a microscope. Separately, similar tissue sections were stained with hematoxylin-eosin. After being deparaffinized, the tissue sections were washed with running tap water for 3 to 5 minutes, and immersed in a Mayer's hematoxylin solution for 5 minutes. The tissue sections were washed with running water at 25 to 37°C for 3 to 5 minutes, and immersed in an eosin solution for 5 minutes. The tissue sections were lightly washed with 60% ethanol, with 70% ethanol, with 80% ethanol and with 95% ethanol, and then immersed in 100% ethanol for 5 minutes 3 times. The tissue sections were immersed in xylene for 5 minutes 3 times, and covered with a cover glass by use of a mounting agent. The glass slide was dried and then observed by a microscope.

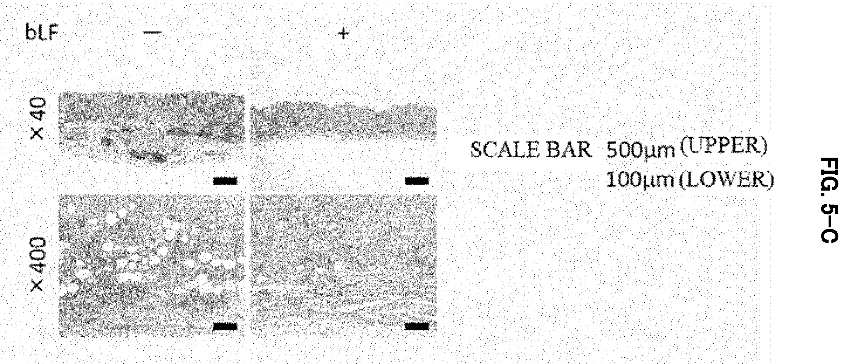

In both of the staining methods, the tissue sections were finally treated with specific esterase staining (chloroacetate esterase) in which only esterase in granules in such tissue sections are stained. After being deparaffinized, the tissue sections were washed with running tap water for 3 to 5 minutes, and washed with distilled water 3 times. The tissue sections were dried at room temperature for 10 to 30 minutes, and immersed in a chloroacetate esterase reaction solution for 15 to 30 minutes. The tissue sections were washed with running tap water for 3 to 5 minutes, and immersed in a Mayer's hematoxylin solution for 5 minutes. The tissue sections were washed with running water at 25 to 37°C for 3 to 5 minutes, and immersed in an eosin solution for 5 minutes. The tissue sections were lightly washed with 60% ethanol, 70% ethanol, 80% ethanol and 95% ethanol, and then immersed in 100% ethanol for 5 minutes 3 times. The tissue sections were immersed in xylene for 5 minutes 3 times, and covered with a cover glass by use of a mounting agent. The glass slide was dried and then observed by a microscope. With the group of mice fed with the lactoferrin-non-containing feed, the subcutaneous bleeding in the top part of muscular layer and the thrombus formation in the blood vessel were significant. By contrast, with the group of mice fed with the lactoferrin-containing feed, the subcutaneous bleeding and the thrombus formation were mild. Neutrophilic infiltration in the subcutaneous tissues was observed to be suppressed by the lactoferrin-containing feed [FIG. 5-C].

### 3. Production LSR model 2 and its evaluation 2

An air pouch was created subcutaneously on the back of each mouse to induce LSR, and the DNA concentration in the air pouch was measured and the neutrophils were observed. First, 5 ml of air was subcutaneously injected vigorously on the back with a 5 ml syringe and a 30 G needle. Three days later, 100 µg of LPS was injected into the air pouch in each mouse. Twenty four hours later, 0.3 µg of TNF-α was injected into the air pouch to each mouse, and 6 hours later, the air pouch was washed with 2 ml of sterilized PBS(-) and recovered.

### 4. Measurement of the DNA concentration in the air pouch (lavage fluid)

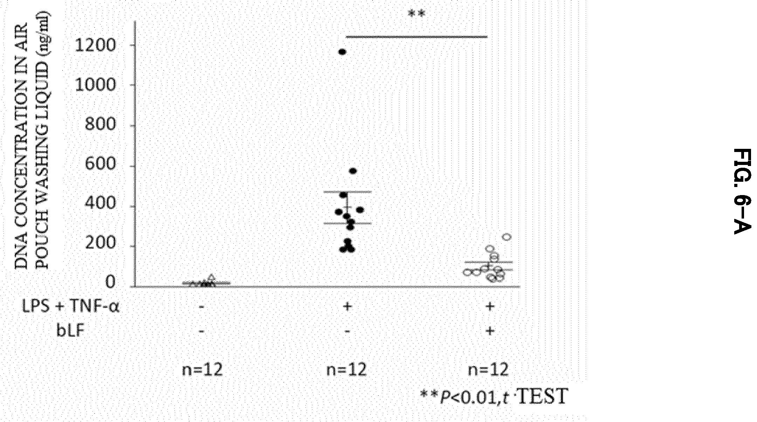

The sampled lavage fluid was transferred to a 1.5 ml microscopic centrifuge tube, and centrifuged at room temperature at 6000 g for 5 minutes. The DNA concentration in the supernatant was measured by use of Picogreen dsDNA assay reagent in accordance with an accompanying protocol. The group of mice fed with the lactoferrin-containing feed had a smaller DNA amount in the blood than the group of mice fed with the lactoferrin-non-containing feed. This indicates that the release of the DNAs by the NETs was significantly suppressed with the group of mice fed with the lactoferrin-containing feed (p = 0.0015) [FIG. 6-A].

### 5. Observation of the formation of the neutrophil NETs

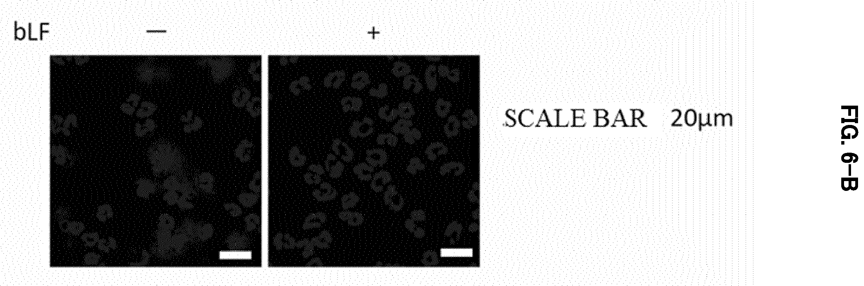

The neutrophils in the lavage fluid were immobilized on a glass slide by use of Cytospin 2 (Shandon), stained with 5 µM DRAQ5, and observed by a microscope. The number of the neutrophils with which the formation of the NETs was observed was smaller with the group of mice the fed with the lactoferrin-containing feed than with the control group of mice fed with the lactoferrin-non-containing feed [FIG. 6-B].

### [Example 6] Improvement effect on the survival rate/lifetime extension of histone-induced thrombus (disseminated intravascular coagulation (DIC)) model mice provided by intravenous injection of lactoferrin to the tails thereof

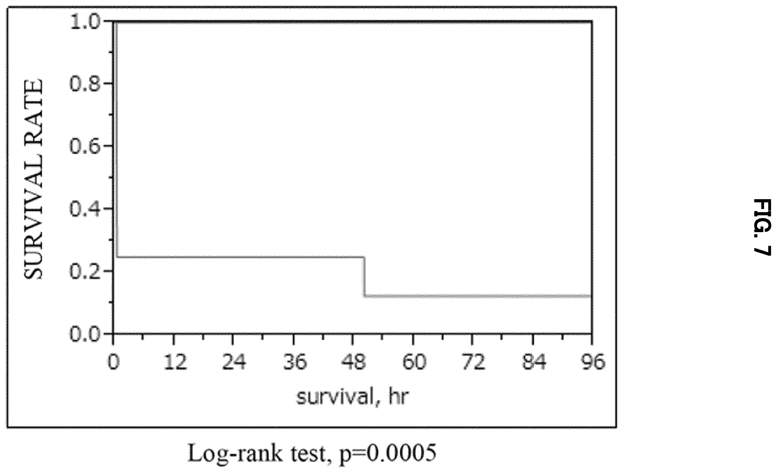

80 mg/kg of histone (sigma, H9250) dissolved in physiological saline and left at 37°C was intravenously administered to the tails of 11 week old male C57BL/6 mice to create DIC model mice (Tobias A. et al., blood, 29 September 2011, vol. 118, no. 13, pp. 3708-3714). As a pre-treatment, 20 mg/kg of bovine lactoferrin was intravenously administered to the tails of the mice, and 30 minute later, histone was intravenously administered to the tails of the mice (n = 8). These mice were labeled as a group of mice provided with therapy with lactoferrin. To the tails of different mice (n = 8), 100 µl of physiological saline not containing bovine lactoferrin was intravenously administered, and 30 minutes later, histone was intravenously administered. These mice were labeled as a control group of mice provided with no therapy. The survival rate/lifetime extension after the administration of histone was evaluated by use of the Kaplan-Meier method. The control group of mice provided with no therapy gradually started to die about 20 minutes after the administration of histone. Two days later, two mice were alive, and 4 days later, one mouse was alive. By contrast, the 8 mice in the group provided with therapy with bovine lactoferrin were all alive even 4 days later. The significant improvement effect on the survival rate/lifetime extension was observed (FIG. 7; statistically significant difference p = 0.0005).

### [Example 7] Effect of hemostasis on histone-induced thrombus model mice (bleeding time duration) provided by intravenous injection of lactoferrin to the tails thereof.

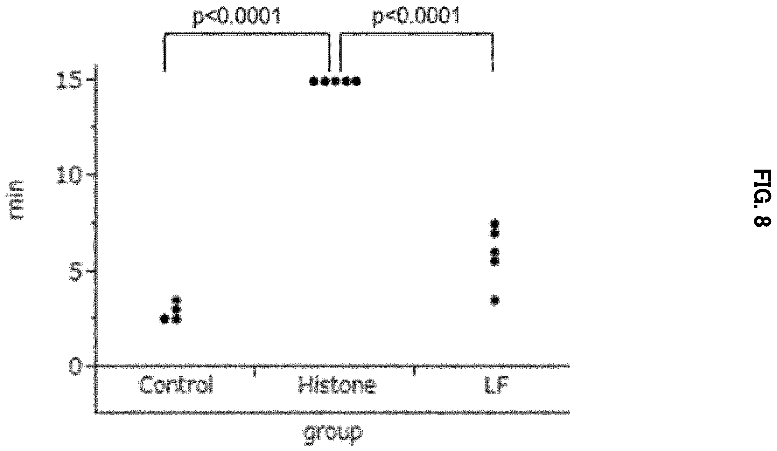

As a pre-treatment, 20 mg/kg of bovine lactoferrin was intravenously administered to the tails of 11 week old male C57BL/6 mice, and 30 minute later, 60 mg/kg of histone (sigma, H9250) dissolved in physiological saline and left at 37°C was intravenously administered to the tails of the mice to check the effect of hemostasis on the DIC model mice (n = 5) (Tobias A, et al., Blood, 2011; 118: pp. 3708-3714) (75% of the amount of histone used for improving the survival rate/lifetime extension was adopted). For control group mice without therapy (n = 5), 100 µl of physiological saline not containing bovine lactoferrin was intravenously administered to the tails 30 minutes before the administration of histone. For healthy control mice (n = 5), physiological saline, instead of the bovine lactoferrin used for the pre-treatment and also instead of histone, was administered. Twenty minutes after the administration of histone, the vein of the tail of each mouse was cut at a position 3 mm from the end, and immersed in a 37°C physiological saline. At every 30 seconds, the blood was wiped off with a filter paper, and the bleeding time duration was measured (in the case where the bleeding time duration was 15 minutes or longer, the bleeding time duration was recorded as 15 minutes and a hemostasis treatment was performed) (Fuchs TA, et al., Blood, 2011; 118:3708-3714). The control group mice provided with no therapy were observed to bleed for a significantly long time (FIG. 8; control group of mice provided with no therapy vs. control group of healthy mice; p < 0.0001). By contrast, with the group of mice provided with therapy with bovine lactoferrin, the bleeding time duration was significantly shorter (FIG. 8; group of mice provided with therapy with bovine lactoferrin vs. control group of mice provided with no therapy; p < 0.0001).

### [Example 8] Suppressing effect of lactoferrin on the bleeding in lung tissues of histone-induced thrombus model mice

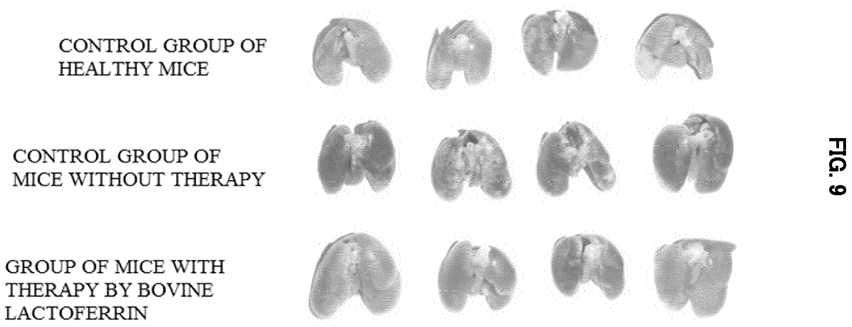

As a pre-treatment, 20 mg/kg of bovine lactoferrin was intravenously administered to the tails of 11 week old male C57BL/6 mice, and 30 minute later, 80 mg/kg of histone (sigma, H9250) dissolved in physiological saline and left at 37°C was intravenously administered to tails of the mice to create DIC model mice (n = 4) (Tobias A., et al., Blood, 29 September 2011; vol. 118: no. 13, pp. 3708-3714). For control group mice with no therapy (n = 4), 100 µl of physiological saline not containing bovine lactoferrin was intravenously administered to the tails 30 minutes before histone was intravenously administered. For healthy control group mice (n = 4), physiological saline, instead of the bovine lactoferrin used for the pre-treatment and also instead of histone, was administered. Ten minutes after the administration of histone (second administration of physiological saline), the mice were put to euthanasia. Lung tissues were extracted and immobilized by use of 4% paraformaldehyde. With the group of mice provided with therapy with bovine lactoferrin, the tissues were not observed to bleed almost at all, unlike with the control group of mice provided with no therapy; and the lung tissues of the group of mice provided with therapy with bovine lactoferrin had a similar color as that of the healthy control group of mice (FIG. 9). With the control group of mice provided with no therapy, the lung tissues were more reddish and were observed to bleed (FIG. 9).

### [Example 9] Experiment to demonstrate that inhibition of the formation of the NETs is a lactoferrin-specific activity

It has been reported that N-terminal fragment of lactoferrin is charged positive and therefore is bindable with a negatively charged DNA molecule (He J, and Furmanski P., Sequence specificity and transcriptional activation in the binding of lactoferrin to DNA. Nature. 1995;373(6516):721-4). Thus, an experiment was performed in order to investigate whether proteins that were of different types from lactoferrin but were close to lactoferrin in the molecular weight (MW) and the isoelectric point (pI) would each inhibit the formation of the NETs. For the experiment, the following proteins were used.

**Table 3**

| Name of protein (bovine) | Molecular weight (kDa) | Isoelectric point |
|---|---|---|
| Lactoferrin | 84.0 | 8.2-8.9 |
| Angiogenin | 14.6 | 9.5 |
| Lactoperoxidase (LPO) | 80.6 | 8.0 |

Angiogenin is known as a tumor angiogenesis factor (Strydom DJ, Fett JW, Lobb RR, Alderman EM, Bethune JL, Riordan JF, and Vallee BL. Amino acid sequence of human tumor derived angiogenin. Biochemistry. 1985; 24:5486-94). Lactoperoxidase is heme peroxidase contained in the milk of mammals at a high concentration like lactoferrin (Sharma S, Singh AK, Kaushik S, Sinha M, Singh RP, Sharma P, Sirohi H, Kaur P, and Singh TP., Lactoperoxidase: structural insights into the function, ligand binding and inhibition. Int J Biochem Mol Biol. 2013; 4:108-28).

### 1. Preparation of lactoperoxidase, angiogenin and lactoferrin

Crude protein isolated from bovine milk was dissolved in 10 mM sodium phosphate buffer (pH 7.0), and the obtained solution was loaded onto an SP-Sepharose column (GE Healthcare Life Sciences) equilibrated with the buffer (flow rate: 3.0 ml/min.). The protein bound to the column was washed with the buffer, and lactoperoxidase, angiogenin and lactoferrin were eluted by the linear concentration gradient of sodium chloride in the buffer (0.3 to 1.0 M).

### 2. Inhibition of the formation of the NETs

The inhibition of the formation of the NETs was performed by measuring the DNA concentration in the culture supernatant in a similar manner to that in Example 2. The experiment was performed triplicate in three independent systems (n = 3).

### 3. Results

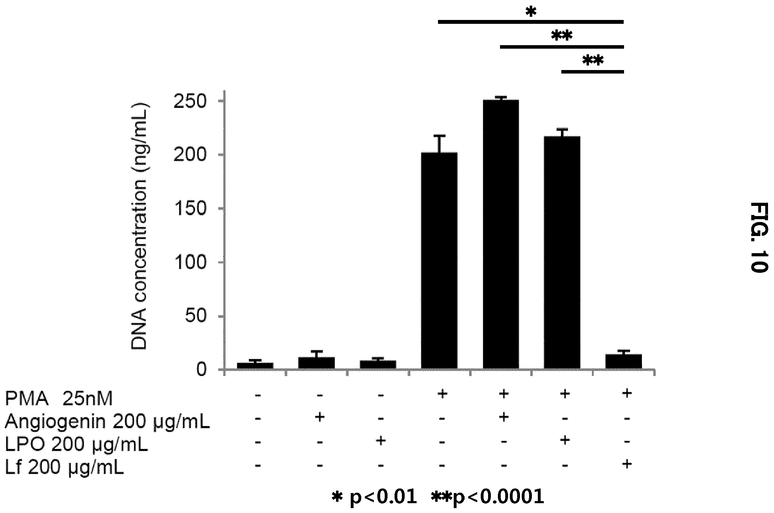

Neither lactoperoxidase nor angiogenin inhibited the formation of the NETs (FIG. 10; p < 0.01). This indicates that the inhibition of the formation of the NETs by lactoferrin is not caused merely by the positive charge of lactoferrin molecules, but is caused by the activity specific to lactoferrin.

### [Description of data presented in the drawings]

FIG. 1 shows the inhibitory effect of lactoferrin added 30 minutes before the neutrophils in the peripheral blood of healthy volunteers were stimulated on the formation of the NETs. (A) In the case where the pre-treatment was performed with 2, 20 or 200 µg/ml of bovine lactoferrin (represented as "bLF" in all the figures), the inhibitory effect on the formation of the NETs was observed in its concentration-dependent manner. In the case where the pre-treatment was performed with 20 µg/ml of bovine lactoferrin, a statistically significant difference of p < 0.01 was obtained. In the case where the pre-treatment was performed with 200 µg/ml of bovine lactoferrin, a statistically significant difference of p < 0.001 was obtained. DPI at 10 µM was used as a positive control. (B) In the case where the pre-treatment was performed with 2, 20 or 200 µg/ml of human lactoferrin (represented as "hLF" in all the figures), the inhibitory effect on the formation of the NETs was observed similarly to the bovine lactoferrin. A statistically significant difference of p < 0.001 was obtained. (C) FIG. 1-C shows images of the pre-treatment performed with 200 µg/ml of human lactoferrin. Extracellular DNAs were stained with 500 nM SYTOX green. It is observed that the release of the DNAs to the outside of the cells is inhibited by the pre-treatment with human lactoferrin. (D) The pre-treatment was performed with human lactoferrin, and the DNA concentration in the culture supernatant 3 hours after the neutrophils were stimulated was measured. The concentration of the DNAs secreted to the outside of the cells by the stimulation performed to form the NETs was suppressed in a bovine-lactoferrin-concentration dependent manner. In the case where the pre-treatment was performed with 20 µg/ml of human lactoferrin, a statistically significant difference of p < 0.05 was obtained. In the case where the pre-treatment was performed with 200 µg/ml of human lactoferrin, a statistically significant difference of p < 0.001 was obtained. (E) The pre-treatment was performed with 200 µg/ml of human lactoferrin, and the neutrophils were analyzed with a scanning electron microscope 3 hours after the neutrophils were stimulated to form the NETs. FIG. 1-E shows images obtained by the scanning electron microscope. In the case where the neutrophils were not stimulated, the neutrophils were kept spherical and the release of the DNAs was not observed. In the case where the neutrophils were stimulated with PMA, the cells were broken and many fiber components were formed. By contrast, the neutrophils pre-treated with human lactoferrin were observed to form such fiber components in bundles. This indicates that the fibers of the NETs were condensed.

FIG. 2 shows the inhibitory effect of lactoferrin on the formation of the NETs added after the neutrophils in the peripheral blood of healthy volunteers were stimulated to form the NETs. The mice pre-treated with 200 µg/ml of human lactoferrin 30 minutes before the neutrophils were stimulated were used as a control group of mice. When 200 µg/ml of lactoferrin was added one or 2 hours after the neutrophils were stimulated to form the NETs, the formation of the NETs was inhibited. (The DNA concentration in the culture supernatant was measured.) In the case where the lactoferrin was added 1 hour after the neutrophils were stimulated, a statistically significant difference of p < 0.001 was obtained. In the case where the lactoferrin was added 2 hours after the neutrophils were stimulated, a statistically significant difference of p < 0.001 was obtained.

FIG. 3 shows the influence of lactoferrin on the survival rate of ANCA model SCG/Kj mice, which are autoimmune disease model mice. At the eighth week in age, the mice were divided into a group of mice fed with the bovine lactoferrin-containing feed (n = 16) and a group of mice fed with the bovine lactoferrin-non-containing feed (n = 16). FIG. 3 shows Kaplan-Meier survival curves up to the 18th week in age. The group of mice fed with the lactoferrin-containing feed exhibited significant improvement in the survival rate. A statistically significant difference of p < 0.05 was obtained.

FIG. 4 shows the influence of administration of lactoferrin on the antibody titer of the MPO-ANCA (myeloperoxidase specific anti-neutrophil cytoplasmic antibody) in the blood, on the DNA in the blood, and on the kidney tissues of the SCG/Kj mice. (A) The SCG/Kj mice, namely, model animals for ANCA associated vasculitis, which is one disease caused by the formation of the NETs, produce MPO-ANCA related to the occurrence of the disease. The antibody titer of the MPO-ANCA of 12 week old SCG/Kj mice was measured by ELISA. With the group of mice fed with the bovine lactoferrin-containing feed (n = 8), the value was lower than with the group of mice fed with the bovine lactoferrin-non-containing feed (n = 8). A statistically significant difference of p < 0.05 was obtained. (B) The DNA content in the blood containing DNAs released from the neutrophils by the formation of the NETs was evaluated. The DNA concentration in the plasma of 13 week old SCG/Kj mice was lower with the group of mice fed with the bovine lactoferrin-containing feed (n = 8) than with the group of mice fed with the bovine lactoferrin-non-containing feed (n = 7). A statistically significant difference of p < 0.05 was obtained. (C) The influence of bovine lactoferrin on the kidney of the SCG/Kj mice was evaluated by use of the Masson trichrome staining. The kidney of the group of mice fed with the bovine lactoferrin-containing feed was milder regarding interstitial fibrosis, inflammatory cell infiltration (upper) and crescent body formation (lower) of the tissues than the kidney of the group of mice fed with the bovine lactoferrin-non-containing feed.

FIG. 5 shows the results of a test regarding the influence, on subcutaneous tissues, of administering lactoferrin to the LSR model 1 mice, which are non-autoimmune disease model mice. (A) Eight week old C57BL/6j mice were divided into a group of mice fed with the bovine lactoferrin-containing feed (n = 16) and a group of mice fed with the bovine lactoferrin-non-containing feed (n = 15). Two weeks later, namely, at the 10th week in age, LPS (100 µg/mouse) and TNFα (0.3 µg/mouse) were subcutaneously administered to the mice to induce rubefaction (subcutaneous bleeding). FIG. 5-A shows images of a part of such mice. With the group of mice fed with the bovine lactoferrin-containing feed, the rubefaction was significantly suppressed in comparison with the group of mice fed with the bovine lactoferrin-non-containing feed. (B) FIG. 5-B is a graph showing the results of quantization performed by use of the evaluation scores on the subcutaneous bleeding shown in Table 2. With the group of mice fed with the bovine lactoferrin-containing feed, the subcutaneous bleeding scores were lower than with the group of mice fed with the bovine lactoferrin-non-containing feed. A statistically significant difference of p < 0.001 was obtained. (C) FIG. 5-C shows the results of evaluation, on the skin tissues of the mice in which LSR was induced, performed by use of the Masson trichrome staining and the esterase staining. With the group of mice fed with the bovine lactoferrin-containing feed, the subcutaneous bleeding and the thrombus formation were suppressed. It is shown by the esterase staining that neutrophil infiltration was suppressed with the group of mice fed with the bovine lactoferrin-containing feed.

FIG. 6 shows the influence, on the air pouch provided subcutaneously on the back, of administering bovine lactoferrin to the LSR model 2 mice, which are non-autoimmune disease model mice. (A) An air pouch was created subcutaneously on the back of each of the mice, and the mice were divided into a group of mice with no inflammation or irritation (n = 12; 100 µg/mouse of LPS; 0.3 µg/mouse of TNF-α), a group of mice fed with the lactoferrin-non-containing feed (n = 12; 100 µg/mouse of LPS; 0.3 µg/mouse of TNF-α), and a group of mice fed with the lactoferrin-containing feed (n = 12). The inside of the air pouches was washed, and the DNA concentration in the washing liquid was measured. With the group of mice fed with the bovine lactoferrin-containing feed, the DNA concentration in the washing liquid was low and close to that with the group of mice with no inflammation or irritation, and a statistically significant difference of p < 0.01 was obtained. (B) FIG. 6-B shows the DNAs, stained with DRAQ5, in the neutrophils in the washing liquid used to wash the air pouches. With the group of mice fed with the bovine lactoferrin-containing feed, the release of the DNAs was scarcely observed at all, unlike with the group of mice fed with the bovine lactoferrin-non-containing feed. This indicates that the formation of the NETs is inhibited with the group of mice fed with the bovine lactoferrin-containing feed.

FIG. 7 shows the results of evaluation on the survival rate/lifetime extension after the administration of histone. The control group of mice with no therapy gradually started to die about 20 minutes after the administration of histone. Two days later, two mice were alive, and 4 days later, one mouse was alive. By contrast, the 8 mice in the group with therapy with bovine lactoferrin were all alive even 4 days later. The significant improvement effect on the survival rate/lifetime extension was observed (statistically significant difference p = 0.0005).

FIG. 8 shows the effect of lactoferrin, which was injected into the tail vein, onhemostasis of histone-induced thrombus model mice. With the control group of mice with no therapy, significantly long bleeding time duration was observed (control group of mice with no therapy vs. control group of healthy mice; p < 0.0001). With the group of mice with therapy with bovine lactoferrin, the bleeding time duration was significantly shorter (group of mice with therapy with bovine lactoferrin vs. control group of mice with no therapy; p < 0.0001).

FIG. 9 shows the suppressing effect of lactoferrin on the bleeding in the lung tissues of the histone-induced thrombus model mice. With the group of mice treated with bovine lactoferrin, no bleeding of tissues was observed almost at all, unlike with the control group with no therapy. The lung tissues of the group of mice treated with bovine lactoferrin showed a similar color as that of the healthy control group of mice. With the control group of mice, the lung tissues were more reddish and were observed to bleed.

FIG. 10 shows that the inhibition of the formation of the NETs is specific activity of lactoferrin. The formation of the NETs was inhibited only by lactoferrin. Neither lactoperoxidase nor angiogenin inhibited the formation of the NETs (FIG. 10; n = 3; p < 0.01).

### INDUSTRIAL APPLICABILITY

The present disclosure provides a therapeutic method with little side effect for a disease caused by the formation of the leukocyte extracellular traps. The method has little side effect and therefore has an advantage of being safely usable for a wide range of patients and people having possibility of becoming patients.

It has been confirmed with non-autoimmune disease model mice that lactoferrin has an ability of inhibiting the formation of the leukocyte extracellular traps. Therefore, according to the present disclosure, lactoferrin exhibits a therapeutic action by a completely novel mechanism that is different from any mechanism of actions reported in the past (Patent document 1).

## Claims

1. A pharmaceutical composition for use in treating a disease associated with formation of leukocyte extracellular traps, the composition comprising lactoferrin, wherein the disease is selected from the group consisting of microscopic polyangitis, allergic granulomatosis-angitis, local Shwartzman reaction, acute kidney injury (AKI) accompanied by ischemia reperfusion injury and disseminated intravascular coagulation.

2. The composition for use according to claim 1, wherein the lactoferrin is a protein selected from the group consisting of:
(a) a protein having the amino acid sequence of any one of SEQ ID NOS: 1 to 5; and
(b) a protein having an amino acid sequence identity of 90% or greater with any one of the amino acid sequences of SEQ ID NOS: 1 to 5, the protein being derived from a mammal and having activity of inhibiting the formation of the leukocyte extracellular traps.

3. The composition for use according to claim 1 or 2, wherein the lactoferrin is prepared in an amount of 0.001 to 10 g/kg/day.

4. The composition for use according to any one of claims 1 to 3, wherein the lactoferrin is derived from human.

5. The composition for use according to any one of claims 1 to 4, wherein the leukocytes are one selected from the group consisting of neutrophils, eosinophil granulocytes, basophil granulocytes, monocytes, macrophages, and mast cells.

6. The composition for use according to any one of claims 1 to 5, which is in a form of an injection agent.

7. The composition for use according to any one of claims 1 to 5, which is orally administrable.

8. The composition for use according to any one of claims 1 to 5, which is in a form of food.

## Patentansprüche

1. Pharmazeutische Zusammensetzung zur Verwendung bei der Behandlung einer mit der Bildung extrazellulärer Fallen von Leukozyten assoziierten Krankheit, wobei die Zusammensetzung Lactoferrin umfasst, wobei die Krankheit aus der aus mikroskopischer Polyangitis, allergischer Granulomatose mit Angitis, lokaler Shwartzman-Reaktion, akuter Nierenverletzung (Acute Kidney Injury, AKI) begleitet von ischämischer Reperfusionsverletzung und disseminierter intravasaler Koagulopathie bestehenden Gruppe ausgewählt ist.

2. Zusammensetzung zur Verwendung nach Anspruch 1, wobei es sich bei dem Lactoferrin um ein Protein ausgewählt aus der Gruppe bestehend aus:
(a) einem Protein mit der Aminosäuresequenz einer der SEQ ID NOS:1 bis 5 und
(b) einem Protein mit einer Aminosäuresequenzidentität von 90% oder mehr mit einer der Aminosäuresequenzen SEQ ID NOS: 1 bis 5, wobei das Protein von einem Säugetier stammt und eine die Bildung der extrazellulären Fallen von Leukozyten hemmende Aktivität aufweist, handelt.

3. Zusammensetzung zur Verwendung nach Anspruch 1 oder 2, wobei das Lactoferrin in einer Menge von 0,001 bis 10 g/kg/Tag produziert wird.

4. Zusammensetzung zur Verwendung nach einem der Ansprüche 1 bis 3, wobei das Lactoferrin vom Menschen stammt.

5. Zusammensetzung zur Verwendung nach einem der Ansprüche 1 bis 4, wobei die Leukozyten aus der aus Neutrophilen, eosinophilen Granulozyten, basophilen Granulozyten, Monozyten, Makrophagen und Mastzellen bestehenden Gruppe ausgewählt sind.

6. Zusammensetzung zur Verwendung nach einem der Ansprüche 1 bis 5, welche in Form eines Injektionsmittels vorliegt.

7. Zusammensetzung zur Verwendung nach einem der Ansprüche 1 bis 5, welche oral verabreichbar ist.

8. Zusammensetzung zur Verwendung nach einem der Ansprüche 1 bis 5, welche in Form eines Nahrungsmittels vorliegt.

## Revendications

1. Composition pharmaceutique pour utilisation dans le traitement d'une maladie associée à la formation de pièges extracellulaires à leucocytes, la composition comprenant de la lactoferrine, la maladie étant choisie dans le groupe constitué de la polyangite microscopique, l'angite allergique granulomateuse, une réaction de Shwartzman locale, une lésion rénale aiguë (LRA) accompagnée d'une lésion d'ischémie-reperfusion et d'une coagulation intravasculaire disséminée.

2. Composition pour utilisation selon la revendication 1, dans laquelle la lactoferrine est une protéine choisie dans le groupe constitué de :
(a) une protéine ayant la séquence d'acides aminés de l'un quelconque de SEQ ID NO: 1 à 5 ; et
(b) une protéine ayant une identité de séquence d'acides aminés de 90 % ou plus avec l'une quelconque des séquences d'acides aminés de SEQ ID NO: 1 à 5, la protéine étant dérivée d'un mammifère et ayant une activité d'inhibition de la formation des pièces extracellulaires à leucocytes.

3. Composition pour utilisation selon la revendication 1 ou 2, dans laquelle la lactoferrine est préparée en une quantité de 0,001 à 10 g/kg/jour.

4. Composition pour utilisation selon l'une quelconque des revendications 1 à 3, dans laquelle la lactoferrine est d'origine humaine.

5. Composition pour utilisation selon l'une quelconque des revendications 1 à 4, dans laquelle les leucocytes sont l'un choisi dans le groupe constitué de neutrophiles, granulocytes éosinophiles, granulocytes basophiles, monocytes, macrophages et mastocytes.

6. Composition pour utilisation selon l'une quelconque des revendications 1 à 5, qui est sous la forme d'un agent d'injection.

7. Composition pour utilisation selon l'une quelconque des revendications 1 à 5, qui est administrable par voie orale.

8. Composition pour utilisation selon l'une quelconque des revendications 1 à 5, qui est sous la forme d'un aliment.
